# EUROPÄISCHE PATENTSCHRIFT

(11) **EP 1 945 634 B1**
(45) Veröffentlichungstag und Bekanntmachung des Hinweises auf die Patenterteilung: **19.01.2011**
(21) Anmeldenummer: 06806430.2
(22) Anmeldetag: 20.10.2006
(51) Int. Cl.: C07D 413/14, A61K 31/422, A61P 7/02

(54) **PHENYLEN-BIS-OXAZOLIDIN-DERIVATE UND IHRE VERWENDUNG ALS ANTIKOAGULANTIEN**
PHENYLENEBISOXAZOLIDINE DERIVATIVES AND THEIR USE AS ANTICOAGULANTS
DERIVES DE PHENYLENE-BIS-OXAZOLIDINE ET LEUR UTILISATION EN TANT QU ANTICOAGULANT

(30) Priorität: 02.11.2005 DE 102005052174
(43) Veröffentlichungstag der Anmeldung: 23.07.2008
(73) Patentinhaber: Bayer Schering Pharma Aktiengesellschaft, 13353 Berlin (DE)
(72) Erfinder: RÖHRIG, Susanne, 45276 Essen (DE); POHLMANN, Jens, CH-4058 Basel (CH); ARNDT, Sabine, 44227 Dortmund (DE); JESKE, Mario, 42699 Solingen (DE); AKBABA, Metin, 40880 Ratingen (DE); PERZBORN, Elisabeth, 42327 Wuppertal (DE); GERDES, Christoph, 51373 Leverkusen (DE); SCHLEMMER, Karl-Heinz, 42113 Wuppertal (DE)
(86) Internationale Anmeldenummer: PCT/EP2006/010129
(87) Internationale Veröffentlichungsnummer: WO 2007/051532

(56) Entgegenhaltungen:
- WO-A1-03/000256
- ZHANG P., ET AL: "Design, synthesis, and SAR of anthranilamide-based factor Xa inhibitors with improved functional activity" BIOORGANIC & MEDICINAL CHEMISTRY LETTERS, Bd. 14, Nr. 4, 2004, Seiten 989-993, XP002414600

## Beschreibung

Die vorliegende Anmeldung betrifft neue 1,4-Phenylen-bis-oxazolidin-Derivate, Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere von thromboembolischen Erkrankungen.

Die Blutgerinnung ist ein Schutzmechanismus des Organismus, mit dessen Hilfe Defekte in der Gefäßwand rasch und zuverlässig "abgedichtet" werden können. So kann ein Blutverlust vermieden bzw. minimiert werden. Die Blutstillung nach Gefäßverletzung erfolgt im wesentlichen durch das Gerinnungssystem, bei dem eine enzymatische Kaskade komplexer Reaktionen von Plasmaproteinen ausgelöst wird. Hierbei sind zahlreiche Blutgerinnungsfaktoren beteiligt, von denen jeder, sobald aktiviert, die jeweils nächste inaktive Vorstufe in ihre aktive Form überführt. Am Ende der Kaskade steht die Umwandlung des löslichen Fibrinogens in das unlösliche Fibrin, so dass es zu einem Blutgerinnsel kommt. Traditionell unterscheidet man bei der Blutgerinnung zwischen dem intrinsischen und dem extrinsischen System, die in einem abschließenden gemeinsamen Reaktionsweg münden. Hierbei kommt dem Faktor Xa, der aus dem Proenzym Faktor X gebildet wird, eine Schlüsselrolle zu, da er beide Gerinnungswege verbindet. Die aktivierte Serinprotease Xa spaltet Prothrombin zu Thrombin. Das entstandene Thrombin wiederum spaltet seinerseits Fibrinogen zu Fibrin. Durch anschließende Quervernetzung der Fibrin-Monomere kommt es zur Bildung von Blutgerinnseln und damit zur Blutstillung. Darüber hinaus ist Thrombin ein potenter Auslöser der Thrombozytenaggregation, die ebenfalls einen erheblichen Beitrag bei der Hämostase leistet.

Die Hämostase unterliegt einem komplexen Regulationsmechanismus. Eine unkontrollierte Aktivierung des Gerinnungssystems oder eine defekte Hemmung der Aktivierungsprozesse kann die Bildung von lokalen Thrombosen oder Embolien in Gefäßen (Arterien, Venen, Lymphgefäßen) oder Herzhöhlen bewirken. Dies kann zu schwerwiegenden thromboembolischen Erkrankungen führen. Darüber hinaus kann eine Hyperkoagulabilität - systemisch - bei einer Verbrauchskoagulopathie zur disseminierten intravasalen Gerinnung führen. Thromboembolische Komplikationen treten ferner auf bei mikroangiopathischen hämolytischen Anämien, extrakorporalen Blutkreisläufen, wie Hämodialyse, sowie Herzklappenprothesen.

Thromboembolische Erkrankungen sind die häufigste Ursache von Morbidität und Mortalität in den meisten industrialisierten Ländern [Heart Disease: A Textbook of Cardiovascular Medicine, Eugene Braunwald, 5. Auflage, 1997, W.B. Saunders Company, Philadelphia].

Die aus dem Stand der Technik bekannten Antikoagulantien, d.h. Stoffe zur Hemmung oder Verhinderung der Blutgerinnung, weisen verschiedene, oftmals gravierende Nachteile auf. Eine effiziente Behandlungsmethode bzw. Prophylaxe von thromboembolischen Erkrankungen erweist sich in der Praxis deshalb als sehr schwierig und unbefriedigend.

Für die Therapie und Prophylaxe von thromboembolischen Erkrankungen findet zum einen Heparin Verwendung, das parenteral oder subkutan appliziert wird. Aufgrund günstigerer pharmakokinetischer Eigenschaften wird zwar heutzutage zunehmend niedermolekulares Heparin bevorzugt; allerdings können auch hierdurch die im folgenden geschilderten bekannten Nachteile nicht vermieden werden, die bei der Therapierung mit Heparin bestehen. So ist Heparin oral unwirksam und besitzt nur eine vergleichsweise geringe Halbwertszeit. Da Heparin gleichzeitig mehrere Faktoren der Blutgerinnungskaskade hemmt, kommt es zu einer unselektiven Wirkung. Darüber hinaus besteht ein hohes Blutungsrisiko, insbesondere können Hirnblutungen und Blutungen im Gastrointestinaltrakt auftreten, und es kann zu Thrombopenie, Alopecia medicomentosa oder Osteoporose kommen [Pschyrembel, Klinisches Wörterbuch, 257. Auflage, 1994, Walter de Gruyter Verlag, Seite 610, Stichwort "Heparin"; Römpp Lexikon Chemie, Version 1.5, 1998, Georg Thieme Verlag Stuttgart, Stichwort "Heparin"].

Eine zweite Klasse von Antikoagulantien stellen die Vitamin K-Antagonisten dar. Hierzu gehören beispielsweise 1,3-Indandione, vor allem aber Verbindungen wie Warfarin, Phenprocoumon, Dicumarol und andere Cumarin-Derivate, die unselektiv die Synthese verschiedener Produkte bestimmter Vitamin K-abhängiger Gerinnungsfaktoren in der Leber hemmen. Durch den Wirkmechanismus bedingt, setzt die Wirkung aber nur sehr langsam ein (Latenzzeit bis zum Wirkeintritt 36 bis 48 Stunden). Die Verbindungen können zwar oral appliziert werden, aufgrund des hohen Blutungsrisikos und des engen therapeutischen Indexes ist aber eine aufwendige individuelle Einstellung und Beobachtung des Patienten notwendig [J. Hirsh, J. Dalen, D.R. Anderson et al., "Oral anticoagulants: Mechanism of action, clinical effectiveness, and optimal therapeutic range" Chest 2001, 119, 8S-21 S; J. Ansell, J. Hirsh, J. Dalen et al., "Managing oral anticoagulant therapy" Chest 2001, 119, 22S-38S; P.S. Wells, A.M. Holbrook, N.R. Crowther et al., "Interactions of warfarin with drugs and food" Ann. Intern. Med. 1994, 121, 676-683].

In jüngster Zeit ist ein neuer Therapieansatz für die Behandlung und Prophylaxe von thromboembolischen Erkrankungen beschrieben worden. Ziel dieses neuen Therapieansatzes ist die Inhibierung von Faktor Xa. Entsprechend der zentralen Rolle, die Faktor Xa in der Blutgerinnungskaskade spielt, stellt Faktor Xa eines der wichtigsten Targets für antikoagulatorische Wirkstoffe dar [J. Hauptmann, J. Stürzebecher, Thrombosis Research 1999, 93, 203; S.A.V. Raghavan, M. Dikshit, "Recent advances in the status and targets of antithrombotic agents" Drugs Fut. 2002, 27, 669-683; H.A. Wieland, V. Laux, D. Kozian, M. Lorenz, "Approaches in anticoagulation: Rationales for target positioning" Curr. Opin. Investig. Drugs 2003, 4, 264-271; U.J. Ries, W. Wienen, "Serine proteases as targets for antithrombotic therapy" Drugs Fut. 2003, 28, 355-370; L.-A. Linkins, J.I. Weitz, "New anticoagulant therapy" Annu. Rev. Med 2005, 56, 63-77 (online-Publikation August 2004)].

Dabei ist gezeigt worden, dass verschiedene, sowohl peptidische wie nicht-peptidische Verbindungen in Tiermodellen als Faktor Xa-Inhibitoren wirksam sind. Eine große Anzahl von direkten Faktor Xa-Inhibitoren ist bislang bekannt [J.M. Walenga, W.P. Jeske, D. Hoppensteadt, J. Fareed, "Factor Xa Inhibitors: Today and beyond" Curr. Opin. Investig. Drugs 2003, 4, 272-281; J. Ruef, H.A. Katus, "New antithrombotic drugs on the horizon" Expert Opin. Investig. Drugs 2003, 12, 781-797; M.L. Quan, J.M. Smallheer, "The race to an orally active Factor Xa inhibitor: Recent advances" Curr. Opin. Drug Discovery & Development 2004, 7, 460-469]. Nicht-peptidische Faktor Xa-Inhibitoren mit Oxazolidinon-Kemstruktur werden in WO 011047919, WO 02/064575 und WO 03/000256 beschrieben.

Die Aufgabe der vorliegenden Erfindung liegt in der Bereitstellung neuer Substanzen zur Bekämpfung von Erkrankungen, insbesondere von thromboembolischen Erkrankungen, die eine verbesserte Löslichkeit in Wasser und physiologischen Medien aufweisen.

Gegenstand der vorliegenden Erfindung sind Verbindungen der allgemeinen Formel (I) 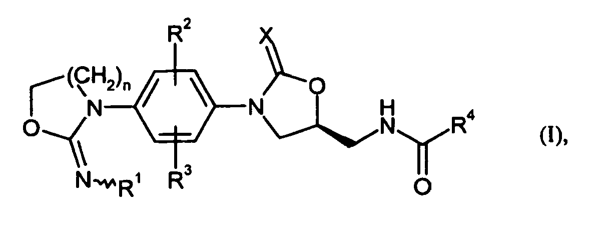 in welcher
- R¹: für Wasserstoff, Hydroxy, Cyano, (C₁-C₆)-Alkyl, (C₁-C₆)-Alkanoyl, Benzoyl oder Hetero- aroyl steht,
- R² und: R³ gleich oder verschieden sind und unabhängig voneinander für Wasserstoff, Fluor, Chlor, Cyano, (C₁-C₄)-Alkyl, Cyclopropyl, Trifluormethyl, Hydroxy, (C₁-C₄)-Alkoxy, Trifluor- methoxy, Amino, Mono- oder Di-(C₁-C₄)-alkylamino stehen,
- R⁴: für Phenyl, Pyridyl, Pyrimidinyl, Pyrazinyl, Thienyl, Furyl oder Pyrrolyl steht, die jeweils ein- oder zweifach, gleich oder verschieden, durch Substituenten ausgewählt aus der Gruppe Halogen Cyano, (C₁-C₄)-Alkyl, welches seinerseits durch Hydroxy oder Amino
substituiert sein kann, (C₁-C₄)-Alkoxy, Ethinyl, Cyclopropyl und Amino substituiert sein können,
- n: für die Zahl 1, 2 oder 3 steht,
und
- X: für O oder N-R⁵ steht, worin -
R⁵ Wasserstoff, Cyano, (C₁-C₆)-Alkyl oder Phenyl bedeutet, wobei Phenyl ein- oder zweifach, gleich oder verschieden, durch Substituenten ausgewählt aus der Gruppe Halogen, Cyano, Trifluormethyl, (C₁-C₄)-Alkyl und (C₁-C₄)-Alkoxy substituiert sein kann,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Erfindung auch Prodrugs der erfindungsgemäßen Verbindungen. Der Begriff "Prodrugs" umfasst Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:

(C₁-C₆-Alkyl und (C₁-C₄)-Alkyl stehen im Rahmen der Ereindung für einen geradkettigen oder verzweigten Alkylrest mit 1 bis 6 bzw. 1 bis 4 Kohlenstoffatomen. Bevorzugt ist ein geradkettiger oder verzweigter Alkylrest mit 1 bis 4 Kohlenstoffatomen. Beispielhaft und vorzugsweise seien genannt: Methyl, Ethyl, n-Propyl, Isopropyl, n-Butyl, iso-Butyl, sec.-Butyl, tert.-Butyl, 1-Ethylpropyl, n-Pentyl und n-Hexyl.

(C₁-C₄)-Alkoxy steht im Rahmen der Erfindung für einen geradkettigen oder verzweigten Alkoxyrest mit 1 bis 4 Kohlenstoffatomen. Beispielhaft und vorzugsweise seien genannt: Methoxy, Ethoxy, n-Propoxy, Isopropoxy, n-Butoxy und tert.-Butoxy.

(C₁-C₆)-Alkanoyl [(C₁-C₆)-Acyl] steht im Rahmen der Erfindung für einen geradkettigen oder verzweigten Alkylrest mit 1 bis 6 Kohlenstoffatomen, der in der 1-Position ein doppelt gebundenes Sauerstoffatom trägt und über die 1-Position verknüpft ist. Bevorzugt ist ein geradkettiger oder verzweigter Alkanoylrest mit 1 bis 4 Kohlenstoffatomen. Beispielhaft und vorzugsweise seien genannt: Formyl, Acetyl, Propionyl, n-Butyryl, iso-Butyryl und Pivaloyl.

Mono-(C₁-C₄)-alkylamino steht im Rahmen der Erfindung für eine Amino-Gruppe mit einem geradkettigen oder verzweigten Alkylsubstituenten, der 1 bis 4 Kohlenstoffatome aufweist. Beispielhaft und vorzugsweise seien genannt: Methylamino, Ethylamino, n-Propylamino, Isopropylamino, n-Butylamino und tert.-Butylamino.

Di-(C₁-C₄)-alkylamino steht im Rahmen der Erfindung für eine Amino-Gruppe mit zwei gleichen oder verschiedenen geradkettigen oder verzweigten Alkylsubstituenten, die jeweils 1 bis 4 Kohlenstoffatome aufweisen. Beispielhaft und vorzugsweise seien genannt: *N,N*-Dimethylamino, *N,N-*Diethylamino, *N*-Ethyl-*N*-methylamino, *N*-Methyl-*N*-n-propylamino, *N*-Isopropyl-*N*-methylamino, *N*-Isopropyl-*N*-n-propylamino, *N*-n-Butyl-*N*-methylamino und *N*-tert.-Butyl-*N*-methylamino.

Heteroaroyl (Heteroarylcarbonyl) steht im Rahmen der Erfindung für einen aromatischen Heterocyclus (Heteroaromaten) mit insgesamt 5 oder 6 Ringatomen und bis zu drei gleichen oder verschiedenen Ring-Heteroatomen aus der Reihe N, O und/oder S, der über eine Carbonylgruppe verknüpft ist. Beispielhaft seien genannt: Furoyl, Pyrroyl, Thienoyl, Pyrazolyl, Imidazoyl, Thiazoyl, Oxazoyl, Isoxazoyl, Isothiazoyl, Triazoyl, Oxadiazoyl, Thiadiazoyl, Pyridinoyl, Pyrimidinoyl, Pyridazinoyl, Pyrazinoyl. Bevorzugt ist ein 5- oder 6-gliedriger Heteroaroyl-Rest mit bis zu zwei Heteroatomen aus der Reihe N, O und/oder S wie beispielsweise Furoyl, Thienoyl, Thiazoyl, Oxazoyl, Isoxazoyl, Isothiazoyl, Pyridinoyl, Pyrimidinoyl, Pyridazinoyl, Pyrazinoyl.

Halogen schließt im Rahmen der Erfindung Fluor, Chlor, Brom und Iod ein. Bevorzugt sind Chlor oder Fluor.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem Substituenten.

Bevorzugt sind Verbindungen der Formel (I), in welcher
- R¹: für Wasserstoff, Hydroxy, Cyano, (C₁-C₆)-Alkyl, (C₁-C₆)-Alkanoyl, Benzoyl oder Hetero- aroyl steht,
- R² und R³: gleich oder verschieden sind und unabhängig voneinander für Wasserstoff, Fluor, Chlor, Cyano, (C₁-C₄)-Alkyl, Cyclopropyl, Trifluormethyl, Hydroxy, (C₁-C₄)-Alkoxy, Trifluor- methoxy, Amino, Mono- oder Di-(C₁-C₄)-alkylamino stehen,
- R⁴: für Phenyl, Pyridyl, Pyrimidinyl, Pyrazinyl, Thienyl, Furyl oder Pyrrolyl steht, die jeweils ein- oder zweifach, gleich oder verschieden, durch Substituenten ausgewählt aus der Gruppe Halogen, Cyano, (C₁-C₄)-Alkyl, welches seinerseits durch Hydroxy oder Amino substituiert sein kann, (C₁-C₄)-Alkoxy, Ethinyl, Cyclopropyl und Amino substituiert sein können,
- n: für die Zahl 1, 2 oder 3 steht,
und
- X: für O oder N-R⁵ steht, worin
R⁵ Wasserstoff, Cyano oder (C₁-C₆)-Alkyl bedeutet,
sowie ihre Salze, Solvate und Solvate der Salze.

Besonders bevorzugt sind Verbindungen der Formel (I), in welcher
- R¹: für Wasserstoff, Hydroxy, Cyano oder Methyl steht,
- R²: für Wasserstoff steht,
- R³: für Wasserstoff, Fluor, Chlor, Cyano, Methyl oder Methoxy steht,
- R⁴: für eine Gruppe der Formel 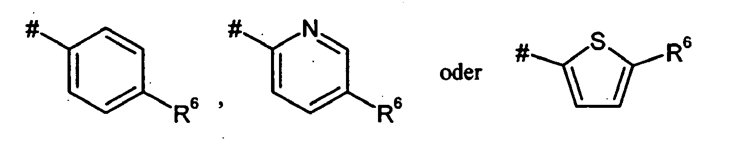 steht, worin
R⁶ Fluor, Chlor, Methyl oder Ethinyl
und
# die Verknüpfungsstelle mit der Carbonylgruppe bedeutet,
- n: für die Zahl 1 oder 2 steht,
und
- X: für O steht,
sowie ihre Salze, Solvate und Solvate der Salze.

Ganz besonders bevorzugt sind Verbindungen der Formel (I), in welcher
- R¹: für Wasserstoff steht,
- R²: für Wasserstoff steht,
- R³: für Wasserstoff, Fluor oder Methyl steht,
- R⁴: für eine Gruppe der Formel 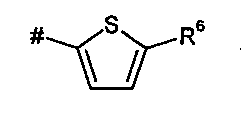 steht, worin
R⁶ Fluor, Chlor oder Methyl
und
# die Verknüpfungsstelle mit der Carbonylgruppe bedeutet,
- n: für die Zahl 1 oder 2 steht,
und
- X: für O steht,
sowie ihre Salze, Solvate und Solvate der Salze.

Bevorzugt sind auch Verbindungen der Formel (I), in welcher R¹ für Wasserstoff steht.

Bevorzugt sind auch Verbindungen der Formel (I), in welcher R² und R³ für Wasserstoff stehen.

Bevorzugt sind auch Verbindungen der Formel (I), in welcher
- R⁴: für eine Gruppe der Formel 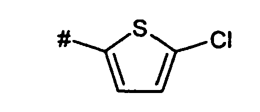
steht, worin # die Verknüpfungsstelle mit der Carbonylgruppe bedeutet.

Bevorzugt sind auch Verbindungen der Formel (I), in welcher X für O steht.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I), in welcher R¹ für Wasserstoff und X für Sauerstoff steht, dadurch gekennzeichnet, dass man Verbindungen der Formel (II) 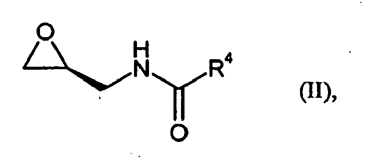 in welcher R⁴ die oben angegebene Bedeutung aufweist,
in einem inerten Lösungsmittel, gegebenenfalls in Gegenwart einer Lewis-Säure, mit einer Verbindung der Formel (III) 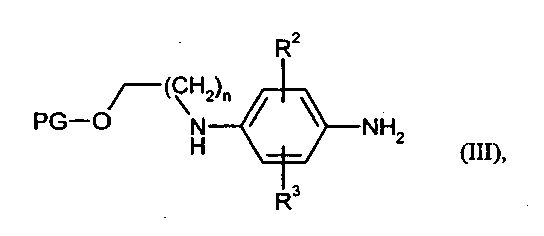 in welcher n, R² und R³ die oben angegebenen Bedeutungen haben
und
- PG: für eine Hydroxy-Schutzgruppe, vorzugsweise für Trimethylsilyl oder *tert*.-Butyldimethyl- silyl, steht,
zu Verbindungen der Formel (IV) 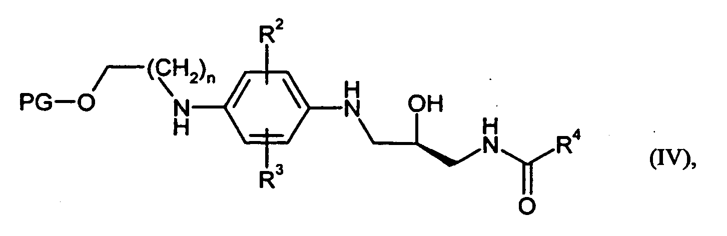 in welcher n, PG, R², R³ und R⁴ die oben angegebenen Bedeutungen aufweisen,
umsetzt, diese dann in einem inerten Lösungsmittel mit einem Kohlensäure-Äquivalent, beispielsweise *N,N'*-Carbonyldimidazol, zu Verbindungen der Formel (V) 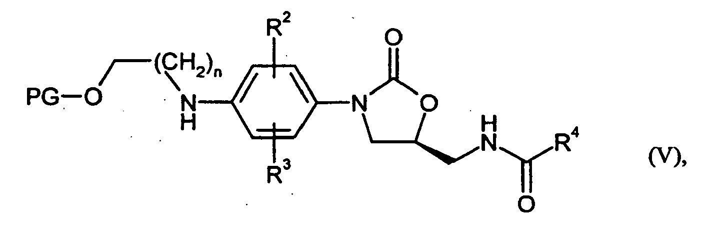 in welcher n, PG, R², R³ und R⁴ die oben angegebenen Bedeutungen aufweisen,
reagiert,
anschließend entweder
[A] durch Abspaltung der Schutzgruppe PG unter üblichen Bedingungen zu Verbindungen der Formel (VI) 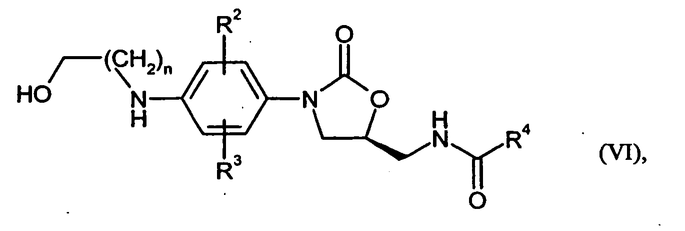 in welcher n, R², R³ und R⁴ die oben angegebenen Bedeutungen haben,
   umsetzt und die Verbindungen der Formel (VI) dann in einem inerten Lösungsmittel in Gegenwart einer Säure mit Bromcyan in Verbindungen der Formel (I-A) 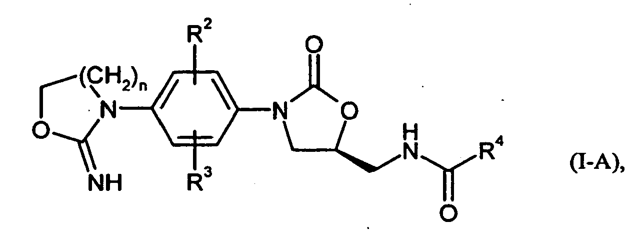 in welcher n, R², R³ und R⁴ die oben angegebenen Bedeutungen haben,
   überführt
   oder
[B] zuerst in einem inerten Lösungsmittel mit Bromcyan, vorzugsweise in Gegenwart einer Base, zu Verbindungen der Formel (VII) 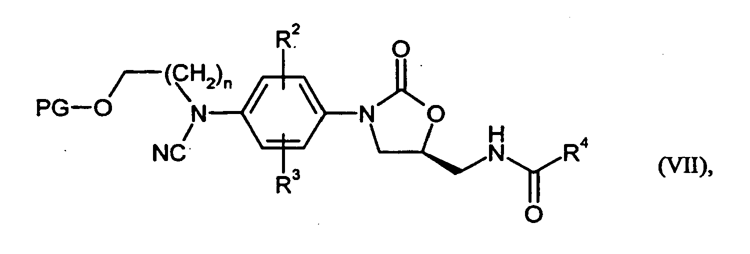 in welcher n, PG, R², R³ und R⁴ die oben angegebenen Bedeutungen haben,
   umsetzt,
   anschließend durch Abspaltung der Schutzgruppe PG in Verbindungen der Formel (VIII) 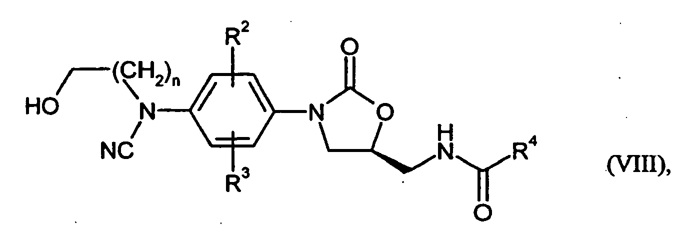 in welcher n, R², R³ und R⁴ die oben angegebenen Bedeutungen haben, überführt und dann die Verbindungen der Formel (VIII) in einem inerten Lösungsmittel in Gegenwart einer Säure zu Verbindungen der Formel (I-A) cyclisiert
   und die Verbindungen der Formel (I-A) gegebenenfalls mit den entsprechenden (i) Lösungsmitteln und/oder (ii) Basen oder Säuren zu ihren Solvaten, Salzen und/oder Solvaten der Salze umsetzt.

Weiterer Gegenstand der Erfindung ist ein Verfahren zur Herstellung der erfindungsgemäßen Verbindungen der Formel (I), in welcher R¹ für Wasserstoff und X für NH steht, dadurch gekennzeichnet, dass man ausgehend von Verbindungen der Formel (IV) zunächst durch erneute Einführung einer Hydroxy-Schutzgruppe PG Verbindungen der Formel (IX) 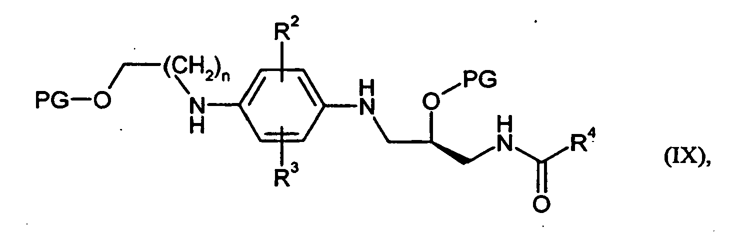 in welcher n, PG, R², R³ und R⁴ die oben angegebenen Bedeutungen aufweisen,
darstellt, diese dann in einem inerten Lösungsmittel mit Bromcyan, vorzugsweise in Gegenwart einer Base, in Verbindungen der Formel (X) 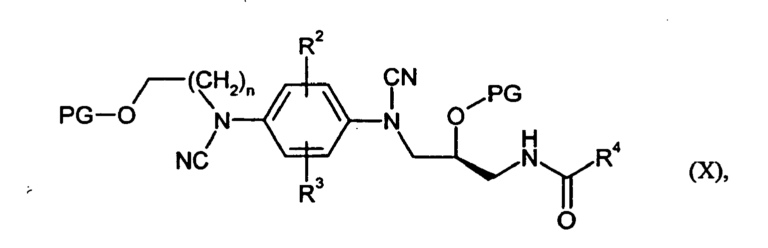 in welcher n, PG, R², R³ und R⁴ die oben angegebenen Bedeutungen haben,
überführt, anschließend durch Abspaltung der Schutzgruppen PG zu Verbindungen der Formel (XI) 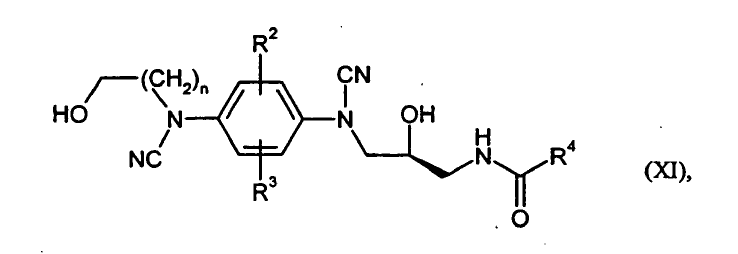 in welcher n, R², R³ und R⁴ die oben angegebenen Bedeutungen haben,
umsetzt und diese in einem inerten Lösungsmittel in Gegenwart einer Säure zu Verbindungen der Formel (I-B) 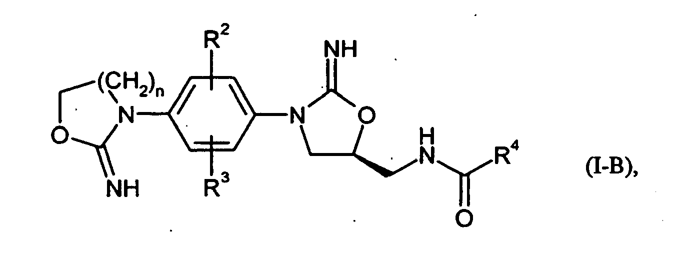 in welcher n, R², R³ und R⁴ die oben angegebenen Bedeutungen haben,
cyclisiert
und die Verbindungen der Formel (I-B) gegebenenfalls mit den entsprechenden (i) Lösungsmittels und/oder (ii) Basen oder Säuren zu ihren Solvaten, Salzen und/oder Solvaten der Salze umsetzt.

Die erfindungsgemäßen Verbindungen der Formel (I), in welcher R¹ nicht für Wasserstoff steht, können ausgehend von den Verbindungen der Formel (VI) in Analogie zu literaturbekannten Verfahren hergestellt werden [vgl. z.B. für R¹ = Alkanoyl: D. Douglass, J. Amer. Chem. Soc. 1934, 56, 719 und T. Shibanuma, M. Shiono, T. Mukaiyama, Chem. Lett. 1977, 575-576; für R¹ = Cyano: a) R. Evers, M. Michalik, J. Prakt. Chem. 1991, 333, 699-710; N. Maezaki, A. Furusawa, S. Uchida, T. Tanaka, Tetrahedron 2001, 57, 9309-9316; G. Berecz, J. Reiter, G. Argay, A. Kalman, J. Heterocycl. Chem. 2002, 39, 319-326; b) R. Mohr, A. Buschauer, W. Schunack, Arch. Pharm. (Weinheim Ger.) 1988, 321, 221-227; für R¹ = Alkyl: a) V.A. Vaillancourt et al., J. Med. Chem. 2001, 44, 1231-1248; b) F.B. Dains et al., J. Amer. Chem. Soc. 1925, 47, 1981-1989; J. Amer. Chem. Soc. 1922, 44, 2637-2643 und T. Shibanuma, M. Shiono, T. Mukaiyma, Chem. Lett. 1977, 575-576; siehe auch Syntheseschemata 3 und 4].

Die erfindungsgemäßen Verbindungen können gegebenenfalls auch durch weitere Umwandlungen von funktionellen Gruppen einzelner Substituenten, insbesondere den unter R², R³ und R⁴ aufgeführten, ausgehend von den nach obigen Verfahren erhaltenen Verbindungen der Formel (I) hergestellt werden. Diese Umwandlungen werden nach üblichen Methoden durchgeführt und umfassen beispielsweise Reaktionen wie Alkylierung, Aminierung, Acylierung, Veresterung, Esterspaltung, Amid-Bildung, Oxidation oder Reduktion sowie die Einführung und Entfernung temporärer Schutzgruppen.

Inerte Lösungsmittel für den Verfahrensschritt (II) + (III) → (IV) sind beispielsweise Alkohole wie Methanol, Ethanol, n-Propanol, iso-Propanol, n-Butanol oder tert.-Butanol, Ether wie Diethylether, Dioxan, Tetrahydrofuran, 2-Methyltetrahydrofuran, Glykoldimethylether oder Diethylenglykoldimethylether, oder andere Lösungsmittel wie Aceton, Dimethylformamid, Dimethylsulfoxid, *N*,*N'*-Dimethylpropylenharnstoff (DMPU), *N*-Methylpyrrolidon (NMP), Acetonitril oder auch Wasser. Ebenso ist es möglich, Gemische der genannten Lösungsmittel einzusetzen. Bevorzugt werden Dioxan, Tetrahydrofuran, Ethanol oder deren Gemische mit Wasser verwendet.

Der Verfahrensschritt (II) + (III) → (IV) kann wahlweise auch unter Zusatz einer katalytischen Menge einer Lewis-Säure, wie beispielsweise Ytterbium(III)trifluormethansulfonat, durchgeführt werden.

Die Umsetzung (II) + (III) → (IV) erfolgt im Allgemeinen in einem Temperaturbereich von 0°C bis +100°C, bevorzugt von +20°C bis +80°C.

Der Ringschluss zum Oxazolidinon im Verfahrensschritt (IV) → (V) erfolgt vorzugsweise mit *N*,*N'*-Carbonyldiimidazol als Kohlensäure-Äquivalent unter Zusatz von 4-*N*,*N*-Dimethylaminopyridin als Base. Die Umsetzung wird bevorzugt in Tetrahydrofuran als Lösungsmittel in einem Temperaturbereich von +20°C bis +70°C durchgerührt.

In den Verfahrensschritten (V) → (VI), (VII) → (VIII) bzw. (X) → (XI) kann die Abspaltung von Trimethylsilyl oder *tert*.-Butyldimethylsilyl als bevorzugt verwendeten Hydroxy-Schutzgruppen (PG) vorzugsweise mit Hilfe von Tetra-n-butylammoniumfluorid (TBAF) oder im Falle der Reaktion (V) → (VI) auch mit Fluorwasserstoff erfolgen. Die Umsetzungen werden im Allgemeinen in Tetrahydrofuran als Lösungsmittel in einem Temperaturbereich von 0°C bis +40°C durchgeführt.

Die Reaktionssequenzen (VII) → (VIII) → (I-A) bzw. (X) → (XI) → (I-B) insgesamt werden besonders bevorzugt unter Verwendung einer säurelabilen Hydroxy-Schutzgruppe, wie beispielsweise Trimethylsilyl oder *tert*.-Butyldimethylsilyl, in Gegenwart eines Überschusses einer Säure als Eintopf-Reaktion, ohne Isolierung der Zwischenstufe (VIII) bzw. (XI), durchgerührt.

Als inerte Lösungsmittel für die Verfahrensschritte (VI) → (I-A), (V) → (VII), (VIII) → (I-A), (IX) → (X) und (XI) → (I-B) eignen sich insbesondere Tetrahydrofuran, Dichlormethan, Acetonitril oder Gemische dieser Lösungsmittel. Diese Verfahrensschritte werden im Allgemeinen in einem Temperaturbereich von -20°C bis +50°C, bevorzugt von 0°C bis +40°C, durchgeführt.

Als Säuren eignen sich bei den Verfahrensschritten (VI) → (I-A) und (VIII) → (I-A) und den Reaktionssequenzen (VII) → (VIII) → (I-A) bzw. (X) → (XI) → (I-B) insbesondere starke anorganische oder organische Säuren wie beispielsweise Fluorwasserstoff, Chlorwasserstoff, Bromwasserstoff, Methansulfonsäure, Trifluormethansulfonsäure oder Trifluoressigsäure.

Die Verfahrensschritte (V) → (VII) und (IX) → (X) werden bevorzugt in Gegenwart einer Base durchgeführt. Hierfür eignen sich insbesondere anorganische Basen wie beispielsweise Alkalioder Erdalkalicarbonate oder -hydrogencarbonate wie Lithium-, Natrium-, Kalium-, Calcium- oder Cäsiumcarbonat oder Natrium- oder Kaliumhydrogencarbonat, oder Alkalihydride wie Natriumhydrid.

Die genannten Umsetzungen können bei normalem, erhöhtem oder bei erniedrigtem Druck durchgeführt werden (z.B. von 0.5 bis 5 bar). Im Allgemeinen arbeitet man jeweils bei Normaldruck.

Die Verbindungen der Formel (II) können nach dem in WO 2004/101557 beschriebenen Verfahren ausgehend von (2S)-3-Aminopropan-1,2-diol der Formel (XII) 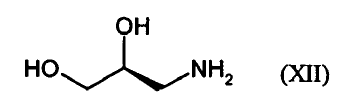 und Carbonsäure-Derivaten der Formel (XIII) 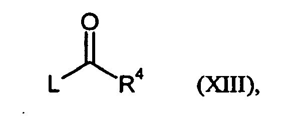 in welcher R⁴ die oben angegebene Bedeutung hat und
- L: für eine Abgangsgruppe wie beispielsweise Halogen, insbesondere für Chlor steht,
hergestellt werden.

Die Verbindungen der Formel (III) können in Analogie zu literaturbekannten Verfahren beispielsweise durch Umsetzung von Verbindungen der Formel (XIV) 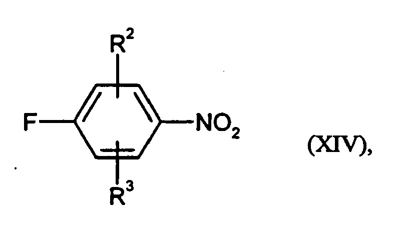 in welcher R² und R³ die oben angegebenen Bedeutungen haben,
mit einer Verbindung der Formel (XV) 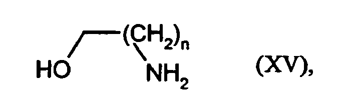 in welcher n die oben angegebene Bedeutung hat,
zu Verbindungen der Formel (XVI) 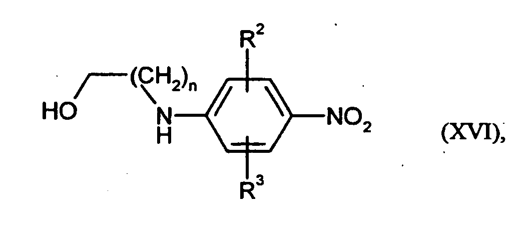 in welcher n, R² und R³ die oben angegebenen Bedeutungen haben,
nachfolgende Einführung der Hydroxy-Schutzgruppe PG und anschließende Reduktion der NitroGruppe zum Amin erhalten werden.

Die Verbindungen der Formeln (XII), (XIII), (XIV) und (XV) sind kommerziell erhältlich, literaturbekannt oder können in Analogie zu literaturbekannten Verfahren hergestellt werden.

Die Herstellung der erfindungsgemäßen Verbindungen kann durch die folgenden Synthese-schemata veranschaulicht werden: 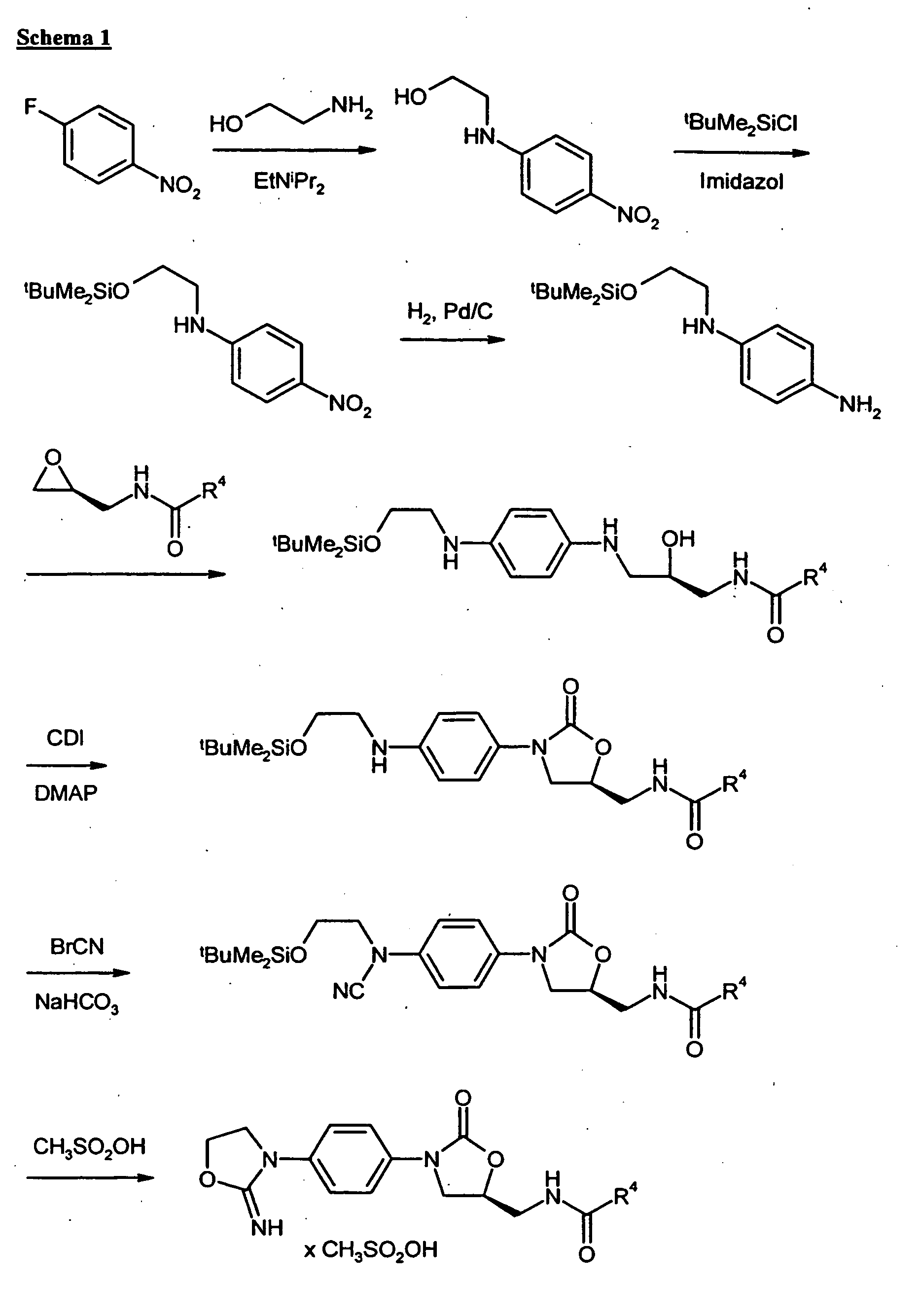 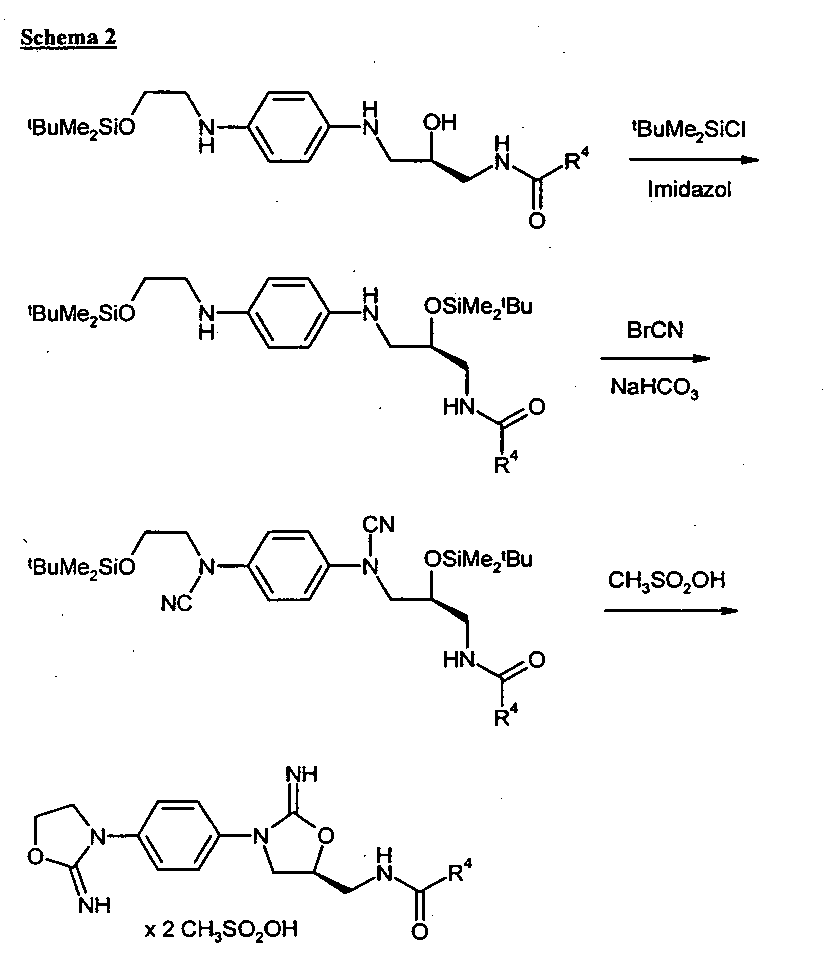 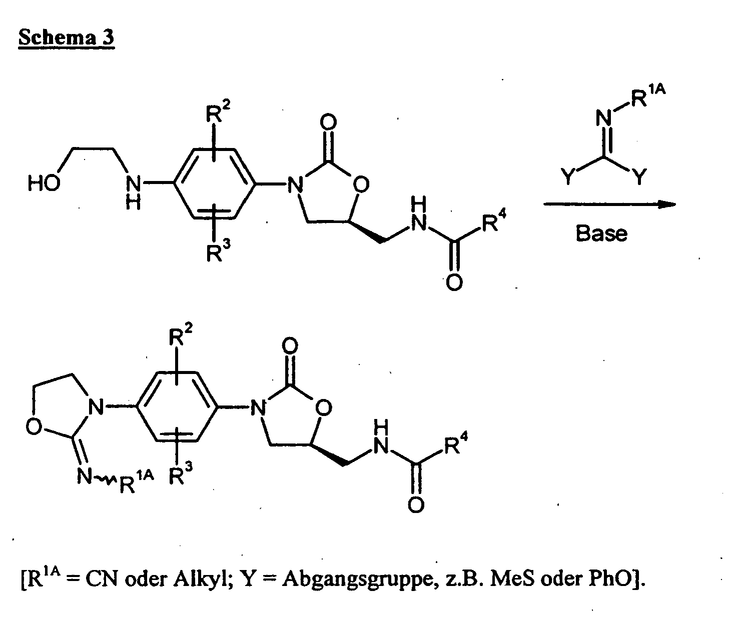 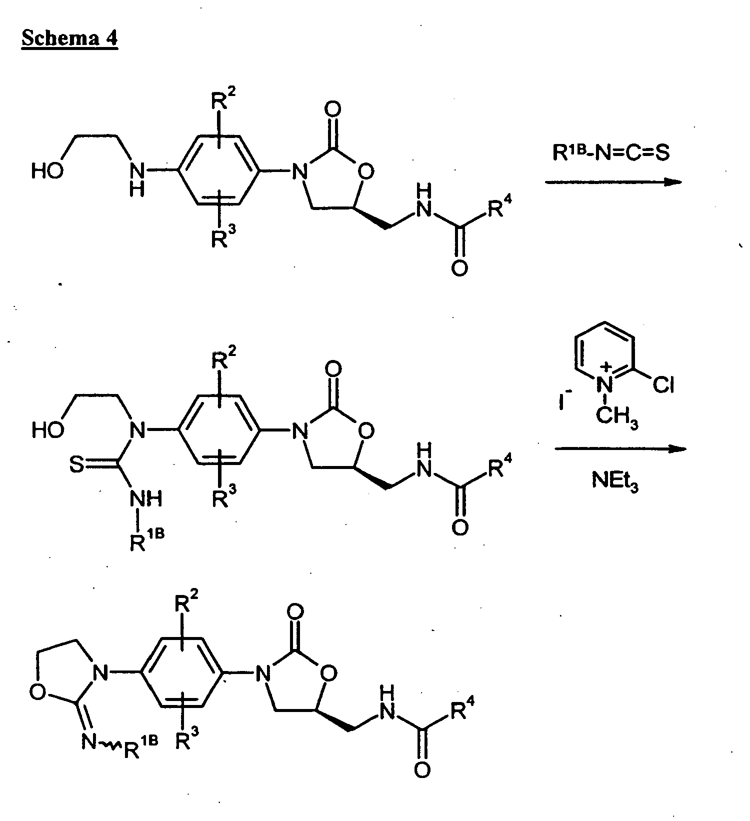 [R^{1B} = Alkyl, Alkanoyl, Benzoyl oder Heteroaroyl].
[Abkürzungen: ^{t}Bu = tert.-Butyl; CDI = *N*,*N*'-Carbonyldiimidazol; DMAP = 4-*N*,*N*-Dimethylaminopyridin; Et = Ethyl; Me = Methyl; Ph = Phenyl; 'Pr = Isopropyl].

Die erfindungsgemäßen Verbindungen zeigen ein nicht vorhersehbares, wertvolles pharmakologisches Wirkspektrum.

Sie eignen sich daher zur Verwendung als Arzneimittel zur Behandlung und/oder Prophylaxe von Krankheiten bei Menschen und Tieren.

Bei den erfindungsgemäßen Verbindungen handelt es sich um selektive Inhibitoren des Blutgerinnungsfaktors Xa, die insbesondere als Antikoagulantien wirken.

Darüber hinaus verfügen die erfindungsgemäßen Verbindungen über günstige physikochemische Eigenschaften, wie beispielsweise eine gute Löslichkeit in Wasser und physiologischen Medien, was für ihre therapeutische Anwendung von Vorteil ist.

Weiterer Gegenstand der vorliegenden Erfindung ist der Einsatz der erfindungsgemäßen Verbindungen zur Behandlung und/oder Prophylaxe von Erkrankungen, vorzugsweise von thromboembolischen Erkrankungen und/oder thromboembolischen Komplikationen.

Zu den "thromboembolischen Erkrankungen" im Sinne der vorliegenden Erfindung zählen insbesondere Erkrankungen wie Herzinfarkt mit ST-Segment-Erhöhung (STEMI) und ohne ST-Segment-Erhöhung (non-STEMI), stabile Angina Pectoris, instabile Angina Pectoris, Reokklusionen und Restenosen nach Koronarinterventionen wie Angioplastie oder aortokoronarem Bypass, periphere arterielle Verschlusskrankheiten, Lungenembolien, tiefe venöse Thrombosen und Nierenvenenthrombosen, transitorische ischämische Attacken sowie thrombotischer und thromboembolischer Hirnschlag.

Die Substanzen eignen sich daher auch zur Prävention und Behandlung von kardiogenen Thromboembolien, wie beispielsweise Hirn-Ischärnien, Schlaganfall und systemischen Thromboembolien und Ischämien, bei Patienten mit akuten, intermittierenden oder persistierenden Herzarrhythmien, wie beispielsweise Vorhofflimmern, und solchen, die sich einer Kardioversion unterziehen, ferner bei Patienten mit Herzklappen-Erkrankungen oder mit künstlichen Herzklappen. Darüber hinaus sind die erfindungsgemäßen Verbindungen zur Behandlung der disseminierten intravasalen Gerinnung (DIC) geeignet.

Thromboembolische Komplikationen treten ferner auf bei mikroangiopathischen hämolytischen Anämien, extrakorporalen Blutkreisläufen, wie Hämodialyse, sowie Herzklappenprothesen.

Außerdem kommen die erfindungsgemäßen Verbindungen auch für die Prophylaxe und/oder Behandlung von atherosklerotischen Gefäßerkrankungen und entzündlichen Erkrankungen wie rheumatische Erkrankungen des Bewegungsapparats in Betracht, darüber hinaus ebenso für die Prophylaxe und/oder Behandlung der Alzheimer'schen Erkrankung. Außerdem können die erfindungsgemäßen Verbindungen zur Inhibition des Tumorwachstums und der Metastasenbildung, bei Mikroangiopathien, altersbedingter Makula-Degeneration, diabetischer Retinopathie, diabetischer Nephropathie und anderen mikrovaskulären Erkrankungen sowie zur Prävention und Behandlung thromboembolischer Komplikationen, wie beispielsweise venöser Thromboembolien, bei Tumorpatienten, insbesondere solchen, die sich größeren chirurgischen Eingriffen oder einer Chemo-oder Radiotherapie unterziehen, eingesetzt werden.

Die erfindungsgemäßen Verbindungen können darüber hinaus auch zur Verhinderung von Koagulation *ex vivo* eingesetzt werden, z.B. zur Konservierung von Blut- und Plasmaprodukten, zur Reinigung/Vorbehandlung von Kathetern und anderen medizinischen Hilfsmitteln und Geräten, zur Beschichtung künstlicher Oberflächen von *in vivo* oder *ex vivo* eingesetzten medizinischen Hilfsmitteln und Geräten oder bei biologischen Proben, die Faktor Xa enthalten.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist ein Verfahren zur Verhinderung der Blutkoagulation *in vitro,* insbesondere bei Blutkonserven oder biologischen Proben, die Faktor Xa enthalten, das dadurch gekennzeichnet ist, dass eine antikoagulatorisch wirksame Menge der erfindungsgemäßen Verbindung zugegeben wird.

Weiterer Gegenstand der vorliegenden Erfindung sind Arzneimittel, enthaltend eine erfindungsgemäße Verbindung und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- Lipidsenker, insbesondere HMG-CoA-(3-Hydroxy-3-methylglutaryl-Coenzym A)-Reduktase-Inhibitoren;
- Koronartherapeutika/Vasodilatatoren, insbesondere ACE-(Angiotensin-Converting-Enzyme)-Inhibitoren; AII-(Angiotensin II)-Rezeptor-Antagonisten; β-Adrenozeptor-Antagonisten; alpha-1-Adrenozeptor-Antagonisten; Diuretika; Calciumkanal-Blocker; Substanzen, die eine Erhöhung von cyclischem Guanosinmonophosphat (cGMP) bewirken, wie beispielsweise Stimulatoren der löslichen Guanylatcyclase;
- Plasminogen-Aktivatoren (Thrombolytika+ibrinolytika) und die Thrombolyse/Fibrinolyse steigernde Verbindungen wie Inhibitoren des Plasminogen-Aktivator-Inhibitors (PAI-Inhibitoren) oder Inhibitoren des Thrombin-aktivierten Fibrinolyse-Inhibitors (TAFI-Inhibitoren);
- antikoagulatorisch wirksame Substanzen (Antikoagulantien);
- plättchenaggregationshemmende Substanzen (Plättchenaggregationshemmer, Thrombozyten• aggregationshemmer);
- Fibrinogen-Rezeptor-Antagonisten (Glycoprotein-IIb/IIIa-Antagonisten);
- sowie Antiarrhythmika.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpynolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- d: Tag(e)
- DMF: *N,N-*Dimethylformamid
- DMSO: Dimethylsulfoxid
- d. Th.: der Theorie (bei Ausbeute)
- eq.: Äquivalent(e)
- ESI: Elektrospray-Ionisation (bei MS)
- h: Stunde(n)
- HPLC: Hochdruck-, Hochleistungsflüssigchromatographie
- LC-MS: Flüssigchromatographie-gekoppelte Massenspektroskopie
- min: Minute(n)
- MS: Massenspektroskopie
- NMR: Kernresonanzspektroskopie
- RP: reverse phase (bei HPLC)
- Rₜ: Retentionszeit (bei HPLC)
- RT: Raumtemperatur
- THF: Tetrahydrofuran

### LC-MS- und HPLC-Methoden:

### Methode 1:

Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 1 1 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min, 2.5 min/3.0 min/4.5 min 2 ml/min; Ofen: 50°C; UV-Detektion: 210 nm.

### Methode 2:

Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min, 2.5 min/3.0 min/4.5 min 2 ml/min; Ofen: 50°C; UV-Detektion: 210 nm.

### Methode 3:

Instrument: Micromass Quattro LCZ mit HPLC Agilent Serie 1100; Säule: Phenomenex Synergi 2µ Hydro-RP Mercury 20 mm x 4 mm; Eluent A: 1 1 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min, 2.5 min/3.0 min/4.5 min 2 ml/min; Ofen: 50°C; UV-Detektion: 208-400 nm.

### Methode 4:

Instrument: Micromass Platform LCZ mit HPLC Agilent Serie 1100; Säule: Phenomenex Synergi 2µ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min, 2.5 min/3.0 min/4.5 min 2 ml/min; Ofen: 50°C; UV-Detektion: 210 nm.

### Methode 5:

Instrument: Micromass Platform LCZ mit HPLC Agilent Serie 1100; Säule: Thermo HyPURITY Aquastar 3µ 50 mm x 2.1 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A → 0.2 min 100% A → 2.9 min 30% A → 3.1 min 10% A → 5.5 min 10% A; Ofen: 50°C; Fluss: 0.8 ml/min; UV-Detektion: 210 nm.

### Methode 6:

Gerätetyp MS: Micromass ZQ; Gerätetyp HPLC: Waters Alliance 2795; Säule: Merck Chromolith SpeedROD RP-18e 50 mm x 4.6 mm; Eluent A: 1 1 Wasser + 0.5 ml 50%-ige Ameisensäure, Eluent B: 11 Acetonitril + 0.5 ml 50%-ige Ameisensäure; Gradient: 0.0 min 10% B → 3.0 min 95% B → 4.0 min 95% B; Ofen: 35°C; Fluss: 0.0 min 1.0 ml/min → 3.0 min 3.0 ml/min → 4.0 min 3.0 ml/min; UV-Detektion: 210 nm.

### Methode 7:

Instrument: HP 1100 mit DAD-Detektion; Säule: Kromasil 100 RP-18, 60 mm x 2.1 mm, 3.5 µm; Eluent A: 5 ml HClO₄ (70%-ig) / 1 Wasser, Eluent B: Acetonitril; Gradient: 0 min 2% B → 0.5 min 2% B → 4.5 min 90% B → 9 min 90% B → 9.2 min 2% B → 10 min 2% B; Fluss: 0.75 ml/min; Säulentemperatur: 30°C; UV-Detektion: 210 nm.

### Methode 8:

Instrument: HP 1100 mit DAD-Detektion; Säule: Kromasil 100 RP-18, 60 mm x 2.1 mm, 3.5 µm; Eluent A: 5 ml HClO₄ (70%-ig) / 1 Wasser, Eluent B: Acetonitril; Gradient: 0 min 2% B → 0.5 min 2% B → 4.5 min 90% B → 15 min 90% B → 15.2 min 2% B → 16 min 2% B; Fluss: 0.75 ml/min; Säulentemperatur: 30°C; UV-Detektion: 210 nm.

### Methode 9:

Instrument: HP 1100 mit DAD-Detektion; Säule: Kromasil 100 RP-18, 60 mm x 2.1 mm, 3.5 µm; Eluent A: 5 ml HClO₄ (70%-ig) / 1 Wasser, Eluent B: Acetonitril; Gradient: 0 min 2% B → 0.5 min 2% B → 4.5 min 90% B → 6.5 min 90% B → 6.7 min 2% B → 7.5 min 2% B; Fluss: 0.75 ml/min; Säulentemperatur: 30°C; UV-Detektion: 210 nm.

### Ausgangsverbindungen und Intermediate:

### Beispiel 1A

### N-(2-{[tert.-Butyl(dimethyl)silyl]oxy}ethyl)benzol-1,4-diamin

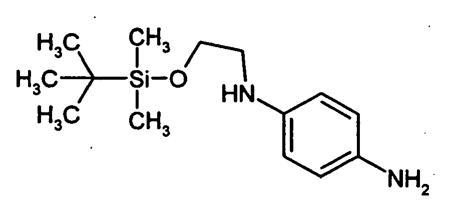

### Stufe a): 2-[(4-Nitrophenyl)amino]ethanol

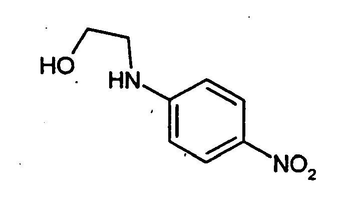

Eine Lösung von 101 g (716 mmol) 4-Fluornitrophenol in 500 ml Ethanol wird mit 130 ml (2.15 mol, 3 eq.) 2-Aminoethanol und 274 ml (1.57 mol, 2.2 eq.) *N*,*N*-Diisopropylethylamin versetzt. Das Reaktionsgemisch wird bei 50°C über Nacht gerührt, anschließend mit weiteren 86 ml (1.43 mol, 2.0 eq.) 2-Aminoethanol und 249 ml (1.43 mol, 2.0 eq.) *N*,*N*-Diisopropylethylamin versetzt und erneut 12 h bei 50°C gerührt. Die Reaktionslösung wird dann im Vakuum eingeengt und der Rückstand mit 600 ml Wasser verrührt. Der entstandene Niederschlag wird abfiltriert, mehrmals mit Wasser gewaschen und getrocknet.
Ausbeute: 127 g (97% d. Th.)
LC-MS (Methode 5): Rₜ = 2.32 min;
MS (ESIpos): m/z= 183 [M+H]⁺;
¹H-NMR (300 MHz, DMSO-d₆): δ = 7.99 (d, 2H), 7.30 (t, 1H), 6.68 (d, 2H), 4.82 (t, 1H), 3.63-3.52 (m, 2H), 3.30-3.19 (m, 2H).

### Stufe b): N-(2-{[tert.-Butyl(dimethyl)silyl]oxy}ethyl)-4-nitroanilin

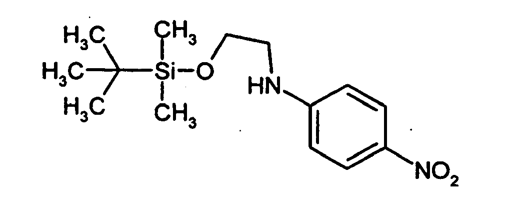

Eine Lösung von 30.8 g (169 mmol) 2-[(4-Nitrophenyl)amino]ethanol in 300 ml DMF wird bei RT mit 30.6 g (203 mmol, 1.2 eq.) *tert*.-Butyldimethylchlorsilan und 17.3 g (254 mmol, 1.5 eq.) Imidazol versetzt und bei RT 2.5 h lang gerührt. Das Reaktionsgemisch wird im Vakuum eingeengt und der Rückstand in 200 ml Dichlormethan und 100 ml Wasser gelöst. Nach Phasentrennung wird die wässrige Phase dreimal mit jeweils 80 ml Dichlormethan extrahiert. Die vereinigten organischen Phasen werden mit 100 ml gesättigter wässriger Natriumchlorid-Lösung gewaschen, über Natriumsulfat getrocknet, filtriert und im Vakuum eingeengt.
Ausbeute: 49.7 g (quant.)
LC-MS (Methode 3): Rₜ = 3.09 min;
MS (ESIpos): m/z = 297 [M+H]⁺;
¹H-NMR (300 MHz, DMSO-d₆): δ = 7.98 (d, 2.H), 7.29 (t, 1H), 6.68 (d, 2H), 3.77-3.66 (m, 2H), 3.35-324 (m, 2H), 0.81 (s, 9H), 0.0 (s, 6H).

### Stufe c): N-(2-{[tert.-Butyl(dimethyl)silyl]oxy}ethyl)benzol-1,4-diamin

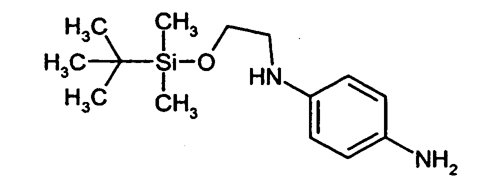

Eine Lösung von 59.5 g (201 mmol) *N*-(2-{[*tert*.-Butyl(dimethyl)silyl]oxy}ethyl)-4-nitroanilin in 500 ml Ethanol wird unter Argon mit 4 g Palladium auf Aktivkohle (10%-ig) versetzt und in einer Wasserstoffatmosphäre bei RT und Normaldruck hydriert. Der Katalysator wird über eine Filterschicht abgetrennt, mit Ethanol gewaschen und das Filtrat im Vakuum eingeengt.
Ausbeute: 53 g (quant.)
LC-MS (Methode 2): Rₜ = 1.83 min;
MS (ESIpos): m/z = 267 [M+H]⁺;
¹H-NMR (300 MHz, DMSO-d₆): δ = 6.42-6.30 (m, 4H), 4.48 (t, 1H), 4.21 (br. s, 2H), 3.68-3.58 (m, 2H), 3.04-2.93 (m, 2H), 0.82 (s, 9H), 0.0 (s, 6H).

### Beispiel 2A

### N-(3-{[tert.-Butyl(dimethyl)silyl]oxy}propyl)benzol-1,4-diamin

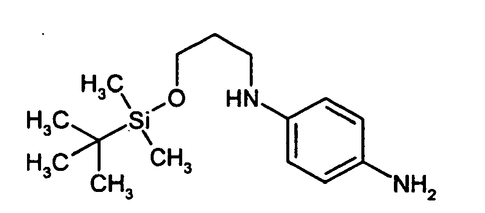

Die Darstellung der Titelverbindung erfolgt durch analoge Reaktionsfolge wie bei Beispiel 1A beschrieben.
LC-MS (Methode 6): Rₜ = 1.73 min;
MS (ESIpos): m/z = 281 [M+H]⁺;
¹H-NMR (400 MHz, DMSO-d₆): δ = 6.39 (d, 2H), 6.30 (d, 2H), 4.56 (br. s, 1H), 4.19 (br. s, 2H), 3.69-3.60 (m, 2H), 2.97-2.88 (m, 2H), 1.70-1.60 (m, 2H), 0.83 (s, 9H), 0.0 (s, 6H).

### Beispiel 3A

### 5-Chlor-N-[(2S)-2-oxiranylmethyl]-2-thiophencarboxamid

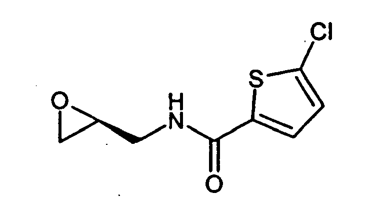

Die Darstellung der Titelverbindung erfolgt wie in WO 2004/101557 (Beispiel 6A) beschrieben durch (*i*) Acylierung von (2*S*)-3-Aminopropan-1,2-diol-Hydrochlorid mit 5-Chlorthiophen-2-carbonsäurechlorid in Gegenwart von Natriumhydrogencarbonat als Base, (*ii*) Hydroxy-Brom-Austausch mit Hilfe von Bromwasserstoffsäure in Essigsäure/Essigsäureanhydrid und (*iii*) Epoxidbildung in Gegenwart von Kaliumcarbonat als Base.

### Ausführungsbeispiele:

### Beispiel 1

### 5-Chlor-N-({(5S)-3-[4-(2-imino-1,3-oxazolidin-3-yl)phenyl]-2-oxo-1,3-oxazolidin-5-yl}methyl)-thiophen-2-carboxamid

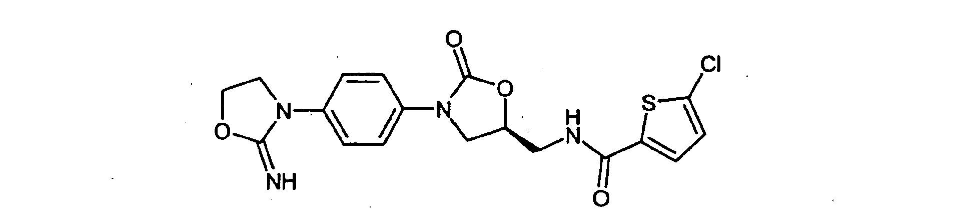

### Stufe a): N-[(2R)-3-({4-[(2-{[tert.-Butyl(dimethyl)silyl]oxy}ethyl)amino]phenyl}amino)-2-hydroxypropyl]-5-chlorthiophen-2-carboxamid

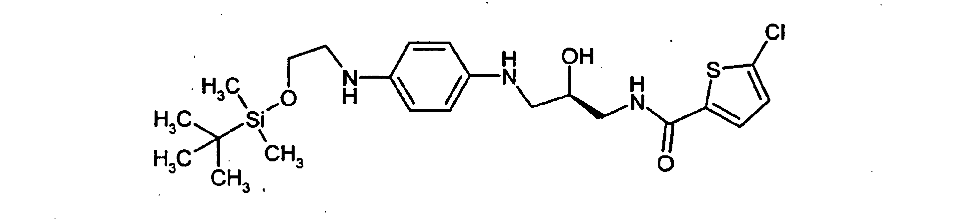

Eine Lösung von 4.6 g (17.3 mmol) der Verbindung aus Beispiel 1A in 50 ml THF wird unter Argon bei Raumtemperatur mit 110 mg (0.17 mmol, 0.01 eq.) Ytterbium(III)trifluormethansulfonat versetzt. Eine Lösung von 2.6 g (12.1 mmol, 0.7 eq.) der Verbindung aus Beispiel 3A in 20 ml THF wird anschließend innerhalb von 2 h bei 60°C zum Reaktionsgemisch getropft. Die Mischung wird über Nacht bei 60°C gerührt, nochmals tropfenweise innerhalb von 1 h mit einer Lösung von 1.1 g (5.2 mmol, 0.3 eq.) der Verbindung aus Beispiel 3A in 10 ml THF versetzt und weitere 2 h bei 60°C gerührt. Das Reaktionsgemisch wird danach im Vakuum eingeengt und das Rohprodukt mittels Flash-Chromatographie an Kieselgel (Laufmittel: Dichlormethan/Methanol 200:1 → 40:1) aufgereinigt.
Ausbeute: 3.7 g (93% Reinheit, 41 % d. Th.)
LC-MS (Methode 2): Rₜ = 2.08 min;
MS (ESI): m/z = 484 [M+H]⁺.

### Stufe b): N-[((5S)-3-{4-[(2-{[tert.-Butyl(dimethyl)silyl]oxy}ethyl)amino]phenyl}-2-oxo-1,3-oxazolidin-5-yl)methyl]-5-chlorthiophen-2-carboxamid

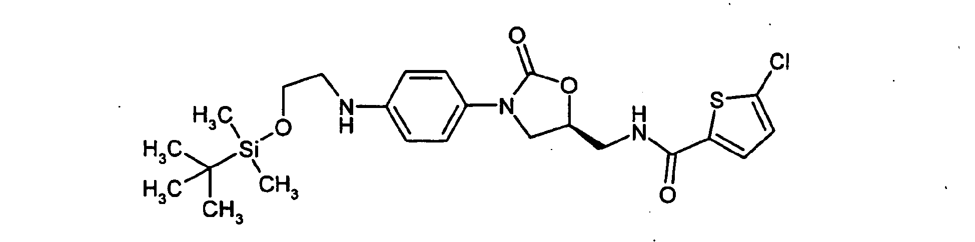

Eine Lösung von 3.7 g (93% Reinheit, 7.1 mmol) *N*-[(2*R*)-3-({4-[(2-{[*tert*.-Butyl(dimethyl)silyl]-oxy}ethyl)amino]phenyl}amino)-2-hydroxypropyl]-5-chlorthiophen-2-carboxamid in 110 ml THF wird unter Argon bei Raumtemperatur mit 400 mg (3.30 mmol, 0.5 eq.) 4-*N*,*N*-Dimethylaminopyridin und 1.6 g (9.9 mmol, 1.4 eq.) *N*,*N*'-Carbonyldiimidazol versetzt. Das Reaktionsgemisch wird bei Raumtemperatur über Nacht gerührt und dann im Vakuum eingeengt. Die Titelverbindung wird mittels Flash-Chromatographie des Rohprodukts an Kieselgel (Laufmittel: Dichlormethan/Methanol 80:1) isoliert.
Ausbeute: 3.6 g (94% Reinheit, 99% d. Th.)
LC-MS (Methode 1): Rₜ = 2.81 min;
MS (ESI): m/z = 510 [M+H]⁺;
¹H-NMR (300 MHz, DMSO-d₆): δ = 8.93 (t, 1H), 7.66 (d, 1H), 7.17 (d, 2H), 7.15 (d, 1H), 6.56 (d, 2H), 5.43 (t, 1H), 4.78-4.67 (m, 1H), 4.03 (t, 1H), 3.70 (dd, 1H), 3.66 (t, 2H), 3.54 (t, 2H), 3.10 (qd, 2H), 0.83 (s, 9H), 0.00 (s, 6H).

### Stufe c): N-[((5S)-3-{4-[(2-{[tert.-Butyl(dimethyl)silyl]oxy}ethyl)(cyano)amino]phenyl}-2-oxo-1,3-oxazolidin-5-yl)methyl]-5-chlorthiophen-2-carboxamid

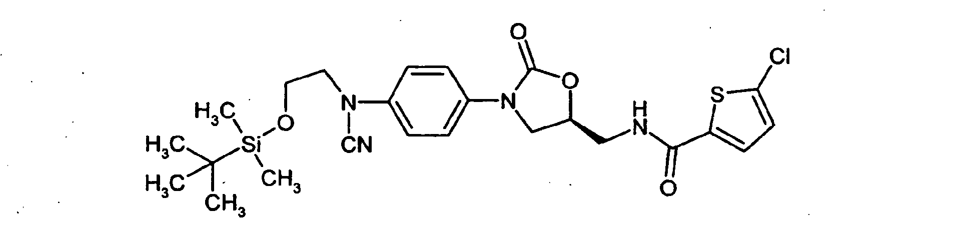

Eine Lösung von 9.3 g (17.3 mmol) *N*-[((5*S*)-3-{4-[(2-{[*tert*.-Butyl(dimethyl)silyl]oxy}ethyl)-amino]phenyl}-2-oxo-1,3-oxazolidin-5-yl)methyl]-5-chlorthiophen-2-carboxamid in 48 ml THF wird unter Argon bei Raumtemperatur mit 4.4 g (51.8 mmol, 3 eq.) Natriumhydrogencarbonat und 6.9 ml Bromcyan-Lösung (3 M in Dichlormethan, 20.7 mmol, 1.2 eq.) versetzt. Das Reaktionsgemisch wird 2 d bei 40°C gerührt und anschließend mit Wasser und Dichlormethan versetzt. Nach Phasentrennung wird die organische Phase mit gesättigter wässriger Natriumhydrogencarbonat-Lösung gewaschen, über Natriumsulfat getrocknet, filtriert und im Vakuum eingeengt. Die Titelverbindung wird durch Verrühren des Rohproduktes in Diisopropylether isoliert.
Ausbeute: 6.8 g (92% Reinheit, 67% d. Th.)
HPLC (Methode 8): Rₜ = 5.26 min;
MS (ESI): m/z = 535 [M+H]⁺;
¹H-NMR (400 MHz, DMSO-d₆): δ = 9.00 (t, 1H), 7.72 (d, 1H), 7.60 (d, 2H), 7.25 (d, 2H), 7.22 (d, 1H), 4.91-4.82 (m, 1H), 4.19 (t, 1H), 3.92-3.81 (m, 5H), 3.67-3.61 (m, 2H), 0.86 (s, 9H), 0.00 (s, 6H).

### Stufe d): 5-Chlor-N-({(5S)-3-[4-(2-imino-1,3-oxazolidin-3-yl)phenyl]-2-oxo-1,3-oxazolidin-5-yl}methyl)thiophen-2-carboxamid

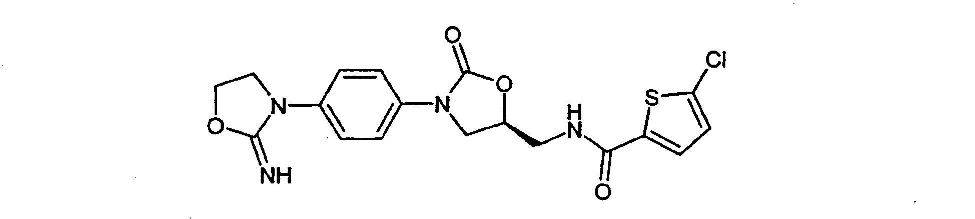

Eine Lösung von 5.4 g (10.1 mmol) *N-*[((5*S*)-3-{4-[(2-{[*tert*.-Butyl(dimethyl)silyl]oxy}ethyl)-(cyano)amino]phenyl}-2-oxo-1,3-oxazolidin-5-yl)methyl]-5-chlorthiophen-2-carboxamid in 100 ml THF wird unter Argon bei Raumtemperatur mit 26 ml Fluorwasserstoffsäure-Lösung (10%-ig in Acetonitril, 101 mmol, 10 eq.) versetzt. Das Reaktionsgemisch wird 2 d bei Raumtemperatur gerührt und dann im Vakuum eingeengt. Der Rückstand wird in 150ml Dichlormethan/Ethanol-Gemisch (15:1) gelöst, mit 10 ml konzentrierter Natriumhydrogencarbonat-Lösung versetzt und 15 min bei Raumtemperatur gerührt. Nach Phasentrennung wird die organische Phase über Magnesiumsulfat getrocknet, filtriert und im Vakuum eingeengt.
Ausbeute: 4.1 g (96% d. Th.)
HPLC (Methode 7): Rₜ = 3.73 min;
MS (ESI): m/z = 421 [M+H]⁺;
¹H-NMR (400 MHz, DMSO-d₆): δ = 8.98 (t, 1H), 7.79 (d, 2H), 7.69 (d, 1H), 7.49 (d, 2H), 7.20 (d, 1H), 6.13 (br. s, 1H), 4.89-4.75 (m, 1H), 4.34 (t, 2H), 4.16 (t, 1H), 3.97 (t, 2H), 3.81 (dd, 1H), 3.60 (t, 2H).

### Beispiel 2

### 5-Chlor-N-({(5S)-3-[4-(2-imino-1,3-oxazolidin-3-yl)phenyl]-2-oxo-1,3-oxazolidin-5-yl}methyl)-thiophen-2-carboxamid-Methansulfonat

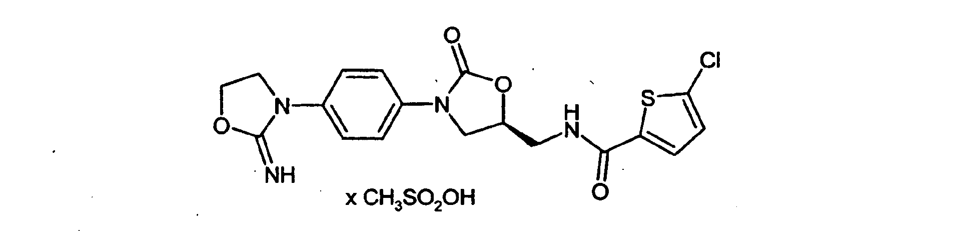

Eine Lösung von 2.0 g (3.7 mmol) *N-*[((5*S*)-3-{4-[(2-{[*tert-*Butyl(dimethyl)silyl]oxy}ethyl)-(cyano)amino]phenyl}-2-oxo-1,3-oxazolidin-5-yl)methyl]-5-chlorthiophen-2-carboxamid (Beispiel 1, Stufe c) in 400 ml Acetonitril wird bei Raumtemperatur mit insgesamt 650 µl (10.1 mmol, 2.7 eq.) Methansulfonsäure versetzt. Das Reaktionsgemisch wird 2 d bei Raumtemperatur gerührt, dann im Vakuum eingeengt und die Titelverbindung mittels Flash-Chromatographie des Rohprodukts an Kieselgel (Laufmittel: Dichlormethan/Methanol 20:1 → 5:1) isoliert.
Ausbeute: 1.9 g (98% d. Th.)
HPLC (Methode 9): Rₜ = 3.71 min;
MS (ESI): m/z = 421 [M+H]⁺ (freie Base);
¹H-NMR (400 MHz, DMSO-d₆): □ = 9.57 (br. s, 1H), 9.00 (t, 1H), 8.80 (br. s, 1H), 7.71 (d, 2H), 7.69 (d, 1H), 7.54 (d, 2H), 7.20 (d, 1H), 4.93-4.85 (m, 1H), 4.84 (t, 2H), 4.22 (t, 3H), 3.86 (dd, 1H), 3.63 (t, 2H), 2.30 (s, 3H).

Die Darstellung der folgenden Salze erfolgt analog zu der in Beispiel 2 beschriebenen Methode durch Umsetzung mit den entsprechenden Säuren:

### Beispiel 3

### 5-Chlor-N-({(5S)-3-[4-(2-imino-1,3-oxazolidin-3-yl)phenyl]-2-oxo-1,3-oxazolidin-5-yl}methyl)-thiophen-2-carboxamid-Trifluoracetat

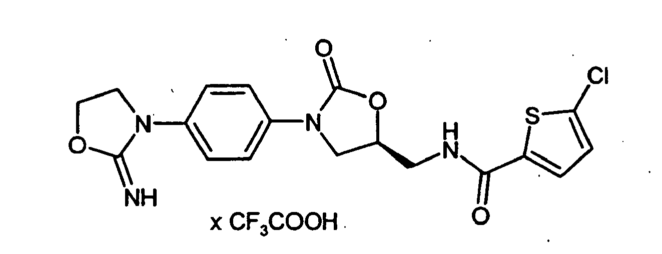

LC-MS (Methode 3): Rₜ =1.30 min;
MS (ESI): m/z = 421 [M+H]⁺ (freie Base);
¹H-NMR (400 MHz, DMSO-d₆): δ = 9.57 (br. s, 1H), 8.99 (t, 1H), 8.79 (br. s, 1H), 7.70 (d, 2H), 7.68 (d, 1H), 7.54 (d, 2H), 7.20 (d, 1H), 4.92-4.84 (m, 1H), 4.84 (t, 2H), 4.21 (t, 3H), 3.86 (dd, 1H), 3.63 (t, 2H).

### Beispiel 4

### 5-Chlor-N-({(5S)-3-[4-(2-imino-1,3-oxazolidin-3-yl)phenyl]-2-oxo-1,3-oxazolidin-5-yl}methyl-) thiophen-2-carboxamid-Hydrochlorid

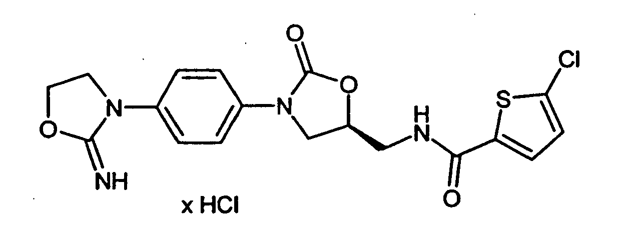

HPLC (Methode 7): Rₜ = 3.73 min;
MS (ESI): m/z = 421 [M+H]⁺ (freie Base);
¹H-NMR (300 MHz, DMSO-d₆): δ = 9.59 (br. s, 1H), 9.07 (t, 1H), 8.81 (br. s, 1H), 7.73 (d,1H), 7.71 (d, 2H), 7.54 (d, 2H), 7.20 (d, 1H), 4.95-4.80 (m, 3H), 4.21 (t, 3H), 3.88 (dd, 1H), 3.62 (t, 2H).

### Beispiel 5

### 5-Chlor-N-({(5S-3-[4-(2-imino-1,3-oxazinan-3-yl)phenyl]-2-oxo-1,3-oxazolidin-5-yl}methyl)-thiophen-2-carboxamid-Methansulfonat

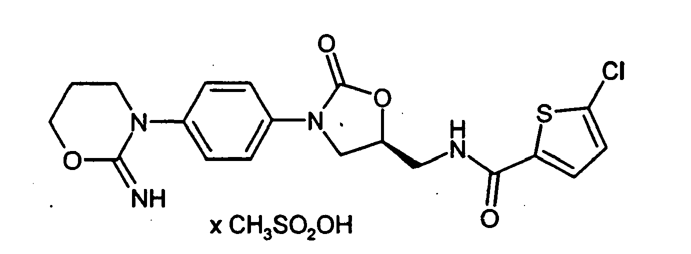

### Stufe a): N-[(2R)-3-({4-[(3-{[tert-Butyl(dimethyl)silyl]oxy}propyl)amino]phenyl}amino)-2-hydroxypropyl]-5-chlorthiophen-2-carboxamid

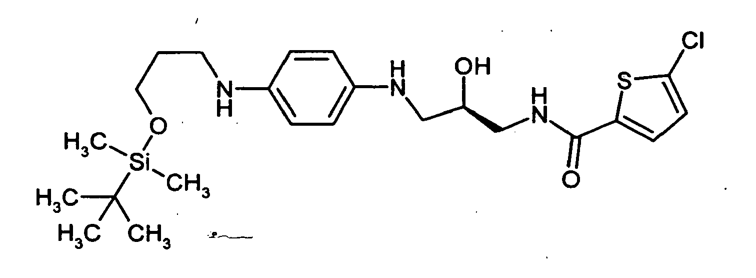

Eine Lösung von 8.6 g (30.6 mmol) der Verbindung aus Beispiel 2A in 40 ml THF wird unter Argon bei Raumtemperatur mit 95 mg (0.15 mmol, 0.005 eq.) Ytterbium(III)trifluormethansulfonat versetzt. Eine Lösung von 4.9 g (22.6 mmol, 0.7 eq.) der Verbindung aus Beispiel 3A in 15 ml THF wird anschließend innerhalb von 2.5 h bei 60°C zum Reaktionsgemisch getropft. Die Mischung wird über Nacht bei 60°C gerührt, nochmals mit 1.7 g (7.9 mmol, 0.3 eq.) der Verbindung aus Beispiel 3A versetzt und weitere 17 h bei 60°C gerührt. Das Reaktionsgemisch wird dann im Vakuum eingeengt und das Rohprodukt mittels Flash-Chromatographie an Kieselgel (Laufmittel: Dichlormethan/Methanol 100:1 → 50:1) aufgereinigt.
Ausbeute: 5.2 g (64% Reinheit, 22% d. Th.)
LC-MS (Methode 1): Rₜ = 1.88 min;
MS (ESI): m/z = 498 [M+H]⁺.

### Stufe b): N-[((5S)-3-{4-[(3-{[tert-Butyl(dimethyl)silyl]oxy}propyl)amino]phenyl}-2-oxo-1,3-oxazolidin-5-yl)methyl]-5-chlorthiophen-2-carboxamid

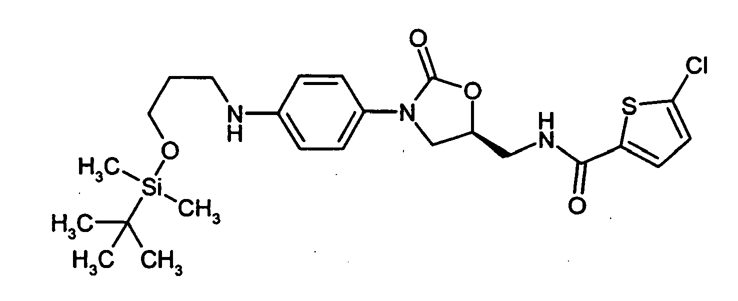

Eine Lösung von 5.2 g (64% Reinheit, 6.70 mmol) *N-*[(2*R*)-3-({4-[(3-{[*tert-*Butyl(dimethyl)silyl]-oxy}propyl)amino]phenyl}amino)-2-hydroxypropyl]-5-chlorthiophen-2-carboxamid in 65 ml THF wird unter Argon bei Raumtemperatur mit 409 mg (3.35 mmol, 0.5 eq.) 4-*N*,*N-*Dimethylaminopyridin und 1.6 g (10.1 mmol, 1.5 eq.) *N*,*N-*Carbonyldiimidazol versetzt. Das Reaktionsgemisch wird 1 d bei Raumtemperatur gerührt, dann mit weiteren 409 mg (3.35 mmol, 0.5 eq.) 4*-N,N-*Dimethylaminopyridin und 1.6 g (10.1 mmol, 1.5 eq.) *N,N'*-Carbonyldiimidazol versetzt und erneut 1 d bei Raumtemperatur gerührt. Das Reaktionsgemisch wird danach im Vakuum eingeengt und die Titelverbindung mittels Flash-Chromatographie des Rohprodukts an Kieselgel (Laufmittel: Dichlormethan/Methanol 300:1 → 100:1) isoliert.
Ausbeute: 1.2 g (98% Reinheit, 33% d. Th.) sowie 620 mg (87% Reinheit, 15% d. Th.)
LC-MS (Methode 1): Rₜ = 2.82 min;
MS (ESI): m/z = 524 [M+H]⁺;
¹H-NMR (300 MHz, DMSO-d₆): δ = 8.94 (t, 1H), 7.66 (d, 1H), 7.17 (d, 1H), 7.15 (d, 2H), 6.51 (d, 2H), 5.49 (t, 1H), 4.77-5.68 (m, 1H), 4.02 (t, 1H), 3.69 (dd, 1H), 3.65 (t, 2H), 3.53 (t, 2H), 2.99 (qd, 2H), 1.68 (q, 2H), 0.84 (s, 9H), 0.00 (s, 6H).

### Stufe c): N-[((5S)-3-{4-[(3-{[tert.-Butyl(dimethyl)silyl]oxy}propyl)(cyano)amino]phenyl}-2-oxo-1,3-oxazolidin-5-yl)methyl]-5-chlorthiophen-2-carboxamid

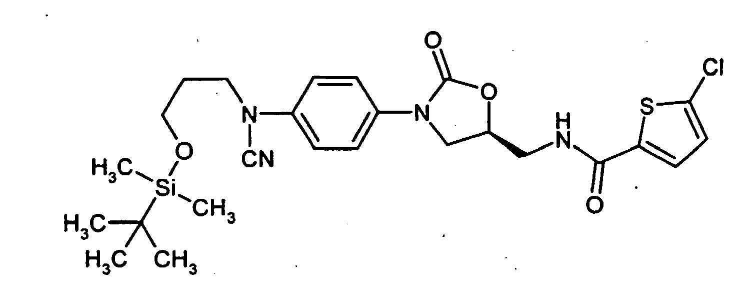

Eine Lösung von 1.2 g (2.2 mmol) *N-*[((5*S*)-3-{4-[(3-{[*tert.*-Butyl(dimethyl)silyl]oxy}propyl}-amino]phenyl}-2-oxo-1,3-oxazolidin-5-yl)methyl]-5-chlorthiophen-2-carboxamid in 7.5 ml THF wird bei Raumtemperatur mit 563 mg (6.70 mmol, 3 eq.) Natriumhydrogencarbonat und 893 µl Bromcyan-Lösung (3 M in Dichlormethan, 2.7 mmol, 1.2 eq.) versetzt. Das Reaktionsgemisch wird 1 d bei 40°C gerührt und anschließend mit Wasser und Dichlormethan versetzt. Nach Phasentrennung wird die organische Phase mit gesättigter wässriger Natriumhydrogencarbonat-Lösung gewaschen, über Natriumsulfat getrocknet, filtriert und im Vakuum eingeengt. Die Titelverbindung wird durch Umkristallisation des Rohprodukts aus Essigsäureethylester isoliert.
Ausbeute: 814 mg (66% d. Th.)
LC-MS (Methode 6): Rₜ = 2.73 min;
MS (ESI): m/z = 549 [M+H]⁺;
¹H-NMR (300 MHz, DMSO-d₆): δ = 8.96 (t, 1H), 7.67 (d, 1H), 7.56 (d, 2H), 7.19 (d, 1H), 7.17 (d, 2H), 4.87-4.76 (m, 1H), 4.15 (t, 1H), 3.81 (dd, 1H), 3.75-3.64 (m, 4H), 3.59 (t, 2H), 1.84 (q, 2H), 0.85 (s, 9H), 0.02 (s, 6H).

### Stufe d): 5-Chlor-N-({(5S)-3-[4-(2-imino-1,3-oxazinan-3-yl)phenyl]-2-oxo-1,3-oxazolidin-5-yl} methyl)thiophen-2-carboxam id-Methansulfonat

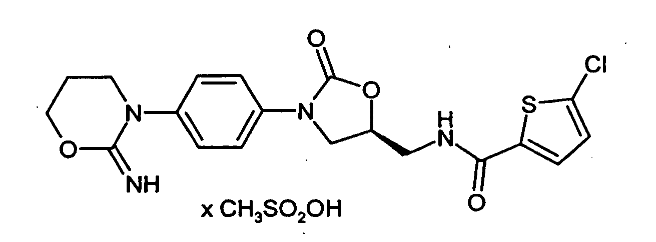

Eine Suspension von 808 mg (1.47 mmol) *N-*[((5*S*)-3-{4-[(3-{[*tert*.-Butyl(dimethyl)silyl]oxy}-propyl)(cyano)amino]phenyl}-2-oxo-1,3-oxazolidin-5-yl)methyl]-5-chlorthiophen-2-carboxamid in 159 ml Acetonitril wird bei Raumtemperatur mit 201 µl (3.09 mmol, 2.1 eq.) Methansulfonsäure versetzt. Das Reaktionsgemisch wird 2 d bei Raumtemperatur gerührt, dann im Vakuum eingeengt und die Titelverbindung mittels Flash-Chromatographie des Rohprodukts an Kieselgel (Laufmittel: Dichlormethan/Methanol 5:1 → 4:1) isoliert.
Ausbeute: 400 mg (51% d. Th.)
HPLC (Methode 7): Rₜ = 3.75 min;
MS (ESI): m/z = 435 [M+H]⁺ (freie Base);
¹H-NMR (400 MHz, DMSO-d₆): δ = 9.00 (t, 1H), 8.74 (br. s, 1H), 7.73 (d, 2H), 7.70 (d, 1H sowie br. s, 1H), 7.55 (d, 2H), 7.20 (d, 1H), 4.94-4.85 (m, 1H), 4.60 (t, 2H), 4.22 (t, 1H), 3.86 (dd, 1H), 3.62 (t, 4H), 2.30 (s, 3H), 2.27 (q, 2H).

### Beispiel 6

### 5-Chlor-N-[((5S)-3-{4-[2-(cyanoimino)-1,3-oxazolidin-3-yl]phenyl}-2-oxo-1,3-oxazolidin-5-yl)-methyl]thiophen-2-carboxamid

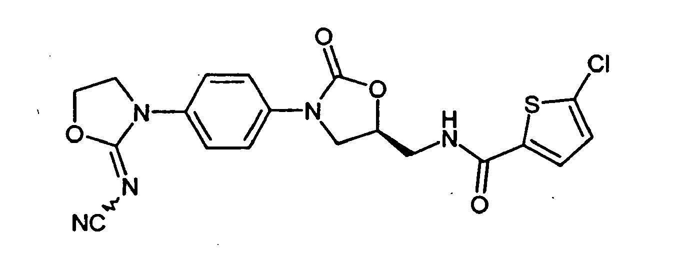

Die Titelverbindung wird als Nebenprodukt erhalten bei der Umsetzung von 5-Chlor-*N-*[((5*S*)-3-{4-[(2-hydroxyethyl)amino]phenyl}-2-oxo-1,3-oxazolidin-5-yl)methyl]thiophen-2-carboxamid (Beispiel 7, Stufe a) mit überschüssiger Bromcyan-Lösung (3 M in Dichlormethan, 50 eq.) in Dichlormethan und anschließende basische Aufarbeitung mit gesättigter wässriger Natriumhydrogencarbonat-Lösung (vgl. Beispiel 1, Stufe c).
HPLC (Methode 7): Rₜ = 3.95 min;
MS (ESI): m/z = 446 [M+H]⁺;
¹H-NMR (400 MHz, DMSO-d₆): δ = 8.97 (t, 1H), 7.68 (d, 1H), 7.63-7.53 (m, 4H), 7.19 (d, 1H), 4.89-4.80 (m, 1H), 4.73 (t, 2H), 4.25 (t, 2H), 4.18 (t, 1H), 3.84 (dd, 1H), 3.61 (t, 2H).

### Beispiel 7

### N-[((5S)-3-{4-[2-(Benzoylimino)-1,3-oxazolidin-3-yl]phenyl}-2-oxo-1,3-oxazolidin-5-yl)methyl]-5-chlorthiophen-2-carboxamid

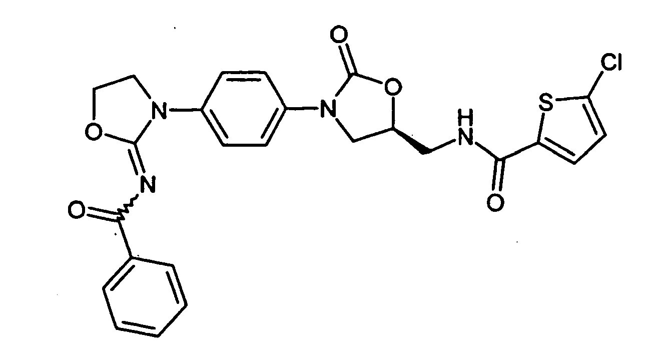

### Stufe a): 5-Chlor-N-[((5S)-3-{4-[(2-hydroxyethyl)amino]phenyl}-2-oxo-1,3-oxazolidin-5-yl)methyl]thiophen-2-carboxamid

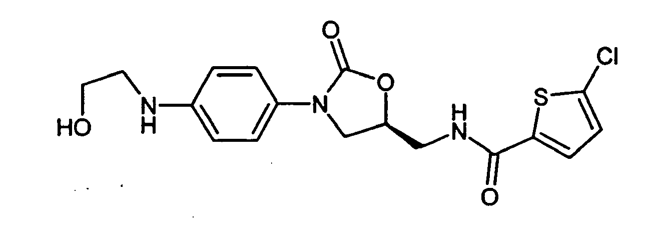

Eine Lösung von 900 mg (1.76 mmol) *N-*[((5*S*)-3-{4-[(2-{[*tert*.-Butyl(dimethyl)silyl]oxy}ethyl)-amino]phenyl}-2-oxo-1,3-oxazolidin-5-yl)methyl]-5-chlorthiophen-2-carboxamid in 25 ml THF wird bei 0°C mit 3.5 ml Tetra-*n*-butylammoniumfluorid-Lösung (1 M in THF, 3.53 mmol, 2 eq.) versetzt. Das Reaktionsgemisch wird 2 h bei Raumtemperatur gerührt und dann im Vakuum eingeengt. Die Titelverbindung wird mittels Flash-Chromatographie des Rohprodukts an Kieselgel (Laufmittel: Dichlormethan/Ethanol 20:1) isoliert.
Ausbeute: 365 mg (52% d. Th.)
LC-MS (Methode 3): Rₜ = 1.62 min;
MS (ESI): m/z = 396 [M+H]⁺;
¹H-NMR (400 MHz, DMSO-d₆): δ = 8.95 (t, 1H), 7.69 (d, 1H), 7.20 (d, 2H), 7.18 (d, 1H), 6.58 (d, 2H), 5.45 (t, 1H), 4.79-4.71 (m, 1H), 4.66 (t, 1H), 4.06 (t, 1H), 3.72 (dd, 1H), 3.57 (t, 2H), 3.53 (qd, 2H), 3.07 (qd, 2H).

### Stufe b): N-[((5S)-3-{4-[[(Benzoylamino)carbonothioyl)(2-hydroxyethyl)amino]phenyl}-2-oxo-1,3-oxazolidin-5-yl)methyl]-5-chlorthiophen-2-carboxamid

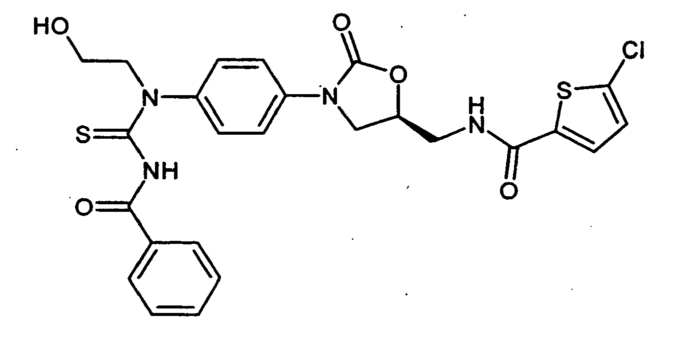

Eine Lösung von 500 mg (1.26 mmol) 5-Chlor-*N-*[((5*S*)-3-{4-[(2-hydroxyethyl)amino]phenyl}-2-oxo-1,3-oxazolidin-5-yl)methyl]thiophen-2-carboxamid in 20 ml Aceton wird unter Argon bei Raumtemperatur mit 200 µl (1.52 mmol, 1.2 eq.) Benzoylisothiocyanat versetzt. Das Reaktionsgemisch wird 3 d bei Raumtemperatur gerührt und dann im Vakuum eingeengt. Die Titelverbindung wird durch Verrühren des Rückstandes in Diisopropylether isoliert.
Ausbeute: 730 mg (86% Reinheit, 89% d. Th.)
HPLC (Methode 7): Rₜ = 4.14 min;
MS (ESI): m/z = 559 [M+H]⁺.

### Stufe c): N-[((5S)-3-{4-[2-(Benzoylimino)-1,3-oxazolidin-3-yl]phenyl)-2-oxo-1,3-oxazolidin-5-yl)methyl]-5-chlorthiophen-2-carboxamid

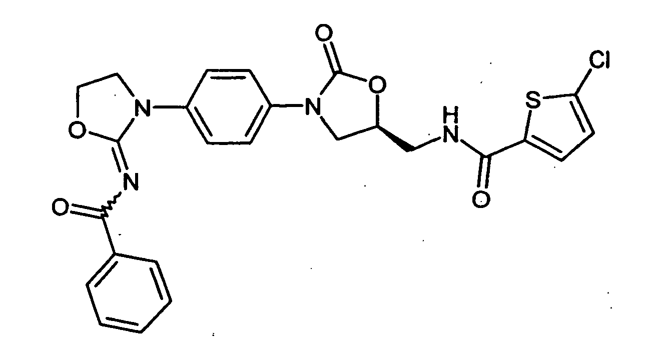

Eine Suspension von 720 mg (1.09 mmol) *N-*[((5*S*)-3-{4-[[(Benzoylamino)carbonothioyl](2-hydroxyethyl)amino]phenyl}-2-oxo-1,3-oxazolidin-5-yl)methyl]-5-chlorthiophen-2-carboxamid und 390 µl (2.73 mmol, 2.5 eq.) Triethylamin in 12 ml Acetonitril wird unter Argon bei Raumtemperatur mit 419 mg (1.64 mmol, 1.5 eq.) 2-Chlor-1-methylpyridiniumiodid versetzt. Das Reaktionsgemisch wird 17 h bei Raumtemperatur gerührt und anschließend mit Wasser und Dichlormethan versetzt. Nach Phasentrennung wird die wässrige Phase mit Dichlormethan nachextrahiert. Die vereinigten organischen Phasen werden mit Wasser gewaschen, über Natriumsulfat getrocknet, filtriert und im Vakuum eingeengt. Die Titelverbindung wird mittels präparativer RP-HPLC isoliert.
Ausbeute: 213 mg (39% d. Th.) sowie 162 mg (90% Reinheit, 26% d. Th.)
LC-MS (Methode 2): Rₜ = 2.34 min;
MS (ESI): m/z = 525 [M+H]⁺;
¹H-NMR (400 MHz, DMSO-d₆): δ = 8.97 (t, 1H), 7.96 (d, 2H), 7.77 (d, 2H), 7.69 (d, 1H), 7.61 (d, 2H), 7.54 (t, 1H), 7.45 (t, 2H), 7.19 (d, 1H), 4.89-4.81 (m, 1H), 4.62 (t, 2H), 4.24-4.15 (m, 3H), 3.86 (dd, 1H), 3.61 (t, 2H).

### B. Bewertung der pharmakologischen Wirksamkeit

Die erfindungsgemäßen Verbindungen wirken insbesondere als selektive Inhibitoren des Blutgerinnungsfaktors Xa und hemmen nicht oder erst bei deutlich höheren Konzentrationen auch andere Serinproteasen wie Plasmin oder Trypsin.

Als "selektiv" werden solche Inhibitoren des Blutgerinnungsfaktors Xa bezeichnet, bei denen die IC₅₀-Werte für die Faktor Xa-Inhibierung gegenüber den IC₅₀-Werten für die Inhibierung anderer Serinproteasen, insbesondere Plasmin und Trypsin, um mindestens das 100-fache kleiner sind, wobei bezüglich der Testmethoden für die Selektivität Bezug genommen wird auf die im folgenden beschriebenen Testmethoden der Beispiele B.a.1) und B.a.2).

Die vorteilhaften pharmakologischen Eigenschaften der erfindungsgemäßen Verbindungen können durch folgende Methoden festgestellt werden:

### a) Testbeschreibungen (in vitro)

### a.1) Messung der Faktor Xa-Hemmung:

Die enzymatische Aktivität von humanem Faktor Xa (FXa) wird über die Umsetzung eines für den FXa-spezifischen chromogenen Substrats gemessen. Dabei spaltet der Faktor Xa aus dem chromogenen Substrat p-Nitroanilin ab. Die Bestimmungen werden wie folgt in Mikrotiterplatten durchgeführt:

Die Prüfsubstanzen werden in unterschiedlichen Konzentrationen in DMSO gelöst und für 10 Minuten mit humanem FXa (0.5 nmol/l gelöst in 50 mmol/l Tris-Puffer [*C,C,C*-Tris(hydroxy-methyl)aminomethan], 150 mmol/l NaCl, 0.1% BSA [bovine serum albumine], pH = 8.3) bei 25°C inkubiert. Als Kontrolle dient reines DMSO. Anschließend wird das chromogene Substrat (150 µmol/l Pefachrome^{®} FXa der Firma Pentapharm) hinzugefügt. Nach 20 Minuten Inkubationsdauer bei 25°C wird die Extinktion bei 405 nm bestimmt. Die Extinktionen der Testansätze mit Prüfsubstanz werden mit den Kontrollansätzen ohne Prüfsubstanz verglichen und daraus die IC₅₀-Werte berechnet.

Repräsentative Wirkdaten aus diesem Test sind in der folgenden Tabelle 1 aufgeführt:

**Tabelle 1**

| **Beispiel Nr.** | **IC₅₀ [nM]** |
|---|---|
| 1 | 1.1 |
| 5 | 3.1 |
| 6 | 16 |

### a.2) Bestimmung der Selektivität:

Zum Nachweis der selektiven FXa-Inhibition werden die Prüfsubstanzen auf ihre Hemmung anderer humaner Serinproteasen wie Trypsin und Plasmin hin untersucht. Zur Bestimmung der enzymatischen Aktivität von Trypsin (500 mU/ml) und Plasmin (3.2 nmol/l) werden diese Enzyme in Tris-Puffen (100 mmol/l, 20 mmol/l CaCl₂, pH = 8.0) gelöst und für 10 Minuten mit Prüfsubstanz oder Lösungsmittel inkubiert. Anschließend wird durch Zugabe der entsprechenden spezifischen chromogenen Substrate (Chromozym Trypsin^{®} und Chromozym Plasmin^{®}; Fa. Roche Diagnostics) die enzymatische Reaktion gestartet und die Extinktion nach 20 Minuten bei 405 nm bestimmt. Alle Bestimmungen werden bei 37°C durchgeführt. Die Extinktionen der Testansätze mit Prüfsubstanz werden mit den Kontrollproben ohne Prüfsubstanz verglichen und daraus die IC₅₀-Werte berechnet.

### a.3) Bestimmung der antikoagulatorischen Wirkung:

Die antikoagulatorische Wirkung der Prüfsubstanzen wird *in vitro* in Human- und Kaninchenplasma bestimmt. Dazu wird Blut unter Verwendung einer 0.11 molaren Natriumcitrat-Lösung als Vorlage in einem Mischungsverhältnis Natriumcitrat/Blut 1:9 abgenommen. Das Blut wird unmittelbar nach der Abnahme gut gemischt und 10 Minuten bei ca. 2500 g zentrifugiert. Der Überstand wird abpipettiert. Die Prothrombinzeit (PT, Synonyme: Thromboplastinzeit, Quick-Test) wird in Gegenwart variierender Konzentrationen an Prüfsubstanz oder dem entsprechenden Lösungsmittel mit einem handelsüblichen Testkit (Hemoliance^{®} RecombiPlastin, Fa. Instrumentation Laboratory) bestimmt. Die Testverbindungen werden 3 Minuten bei 37°C mit dem Plasma inkubiert. Anschließend wird durch Zugabe von Thromboplastin die Gerinnung ausgelöst und der Zeitpunkt des Gerinnungseintritts bestimmt. Es wird die Konzentration an Prüfsubstanz ermittelt, die eine Verdoppelung der Prothrombinzeit bewirkt.

### b) Bestimmung der antithrombotischen Wirkung (in vivo)

### b.1) Arteriovenöses Shunt-Modell (Kaninchen):

Nüchterne Kaninchen (Stamm: Esd: NZW) werden durch intramuskuläre Gabe einer Rompun/Ketavet-Lösung narkotisiert (5 mg/kg bzw. 40 mg/kg). Die Thrombusbildung wird in einem arteriovenösen Shunt in Anlehnung an die von C.N. Berry et al. [Semin. Thromb. Hemost. 1996, 22, 233-241] beschriebene Methode ausgelöst. Dazu werden die linke Vena jugularis und die rechte Arteria carotis freipräpariert. Ein extracorporaler Shunt wird mittels eines 10 cm langen Venenkatheders zwischen den beiden Gefäßen gelegt. Dieser Katheder ist in der Mitte in einen weiteren, 4 cm langen Polyethylenschlauch (PE 160, Becton Dickenson), der zur Erzeugung einer thrombogenen Oberfläche einen aufgerauhten und zu einer Schlinge gelegten Nylonfaden enthält, eingebunden. Der extrakorporale Kreislauf wird 15 Minuten lang aufrechterhalten. Dann wird der Shunt entfernt und der Nylonfaden mit dem Thrombus sofort gewogen. Das Leergewicht des Nylonfadens ist vor Versuchsbeginn ermittelt worden. Die Prüfsubstanzen werden vor Anlegung des extrakorporalen Kreislaufs entweder intravenös über die eine Ohrvene oder oral mittels Schlundsonde verabreicht.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 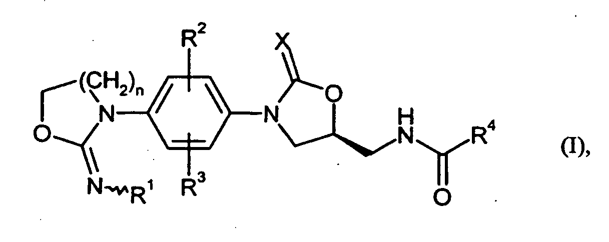 in welcher
R¹ für Wasserstoff, Hydroxy, Cyano, (C₁-C₆₎-Alkyl, (C₁-C₆₎Alkanoyl, Benzoyl oder Heteroaroyl steht,
R² und R³ gleich oder verschieden sind und unabhängig voneinander für Wasserstoff, Flu- or, Chlor, Cyano, (C₁-C₄)-Alkyl, Cyclopropyl, Trifluormethyl, Hydroxy, (C₁-C₄₎- Alkoxy, Trifluormethoxy, Amino, Mono- oder Di-(C₁-C₄)-alkylamino stehen,
R⁴ für Phenyl, Pyridyl, Pyrimidinyl, Pyrazinyl, Thienyl, Furyl oder Pyrrolyl steht, die jeweils ein- oder zweifach, gleich oder verschieden, durch Substituenten ausge- wählt aus der Gruppe Halogen, Cyano, (C₁-C₄)-Alkyl, welches seinerseits durch Hydroxy oder Amino substituiert sein kann, (C₁-C₄)-Alkoxy, Ethinyl, Cyclopropyl und Amino substituiert sein können,
n für die Zahl 1, 2 oder 3 steht,
und
X für O oder N-R⁵ steht, worin
R⁵ Wasserstoff, Cyano, (C₁-C₆)-Alkyl oder Phenyl bedeutet, wobei Phenyl ein- oder zweifach, gleich oder verschieden, durch Substituenten ausgewählt aus der Gruppe Halogen, Cyano, Trifluormethyl, (C₁-C₄)-Alkyl und (C₁-C₄)-Alkoxy substituiert sein kann,
sowie ihre Salze, Solvate und Solvate der Salze.

2. Verbindung der Formel (I) nach Anspruch 1, in welcher
R¹ für Wasserstoff, Hydroxy, Cyano, (C₁-C₆)-Alkyl, (C₁-C₆)-Alkanoyl, Benzoyl oder Heteroaroyl steht,
R² und R³ gleich oder verschieden sind und unabhängig voneinander für Wasserstoff, Flu- or, Chlor, Cyano, (C₁-C₄)-Alkyl, Cyclopropyl, Trifluormethyl, Hydroxy, (C₁-C₄)- Alkoxy, Trifluormethoxy, Amino, Mono- oder Di-(C₁-C₄)-alkylamino stehen,
R⁴ für Phenyl, Pyridyl, Pyrimidinyl, Pyrazinyl, Thienyl, Furyl oder Pyrrolyl steht, die jeweils ein- oder zweifach, gleich oder verschieden, durch Substituenten ausge- wählt aus der Gruppe Halogen, Cyano, (C₁-C₄)-Alkyl, welches seinerseits durch Hydroxy oder Amino substituiert sein kann, (C₁-C₄)-Alkoxy, Ethinyl, Cyclopropyl und Amino substituiert sein können,
n für die Zahl 1, 2 oder 3 steht,
und
X für O oder N-R⁵ steht, worin
R⁵ Wasserstoff, Cyano oder (C₁-C₆)-Alkyl bedeutet,
sowie ihre Salze, Solvate und Solvate der Salze.

3. Verbindung der Formel (I) nach einem der Ansprüche 1 oder 2, in welcher
R¹ für Wasserstoff, Hydroxy, Cyano oder Methyl steht,
R² für Wasserstoff steht,
R³ für Wasserstoff, Fluor, Chlor, Cyano, Methyl oder Methoxy steht,
R⁴ für eine Gruppe der Formel 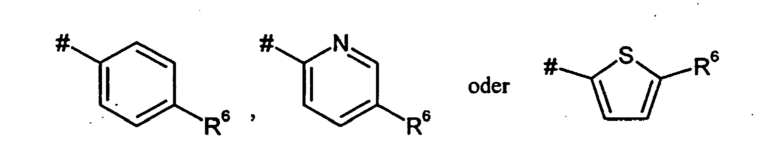 steht, worin
R⁶ Fluor, Chlor, Methyl oder Ethinyl
und
# die Verknüpfungsstelle mit der Carbonylgruppe bedeutet,
n für die Zahl 1 oder 2 steht,
und
X für O steht,
sowie ihre Salze, Solvate und Solvate der Salze.

4. Verbindung der Formel (I) nach einem der Ansprüche 1 bis 3, in welcher
R¹ für Wasserstoff steht,
R² für Wasserstoff steht,
R³ für Wasserstoff, Fluor oder Methyl steht,
R⁴ für eine Gruppe der Formel 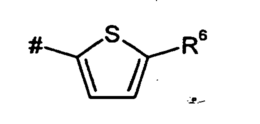 steht, worin
R⁶ Fluor, Chlor oder Methyl
und
# die Verknüpfungsstelle mit der Carbonylgruppe bedeutet,
n für die Zahl 1 oder 2 steht,
und
X für O steht,
sowie ihre Salze, Solvate und Solvate der Salze.

5. Verfahren zur Herstellung von Verbindungen der Formel (I), wie in Anspruch 1 definiert, in welcher R¹ für Wasserstoff und X für Sauerstoff steht, **dadurch gekennzeichnet, dass** man Verbindungen der Formel (II) 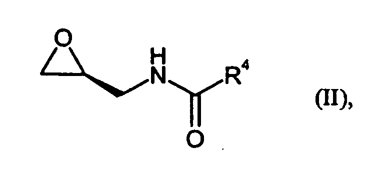 in welcher R⁴die in Anspruch 1 angegebene Bedeutung aufweist,
in einem inerten Lösungsmittel mit einer Verbindung der Formel (III) 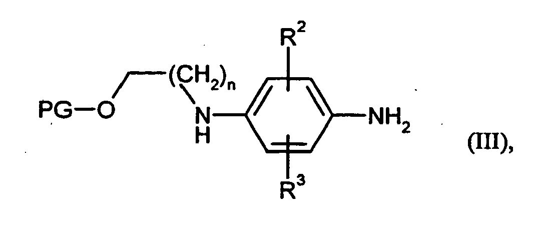 in welcher n, R² und R³ die in Anspruch 1 angegebenen Bedeutungen haben
und
PG für eine Hydroxy-Schutzgruppe steht,
zu Verbindungen der Formel (IV) 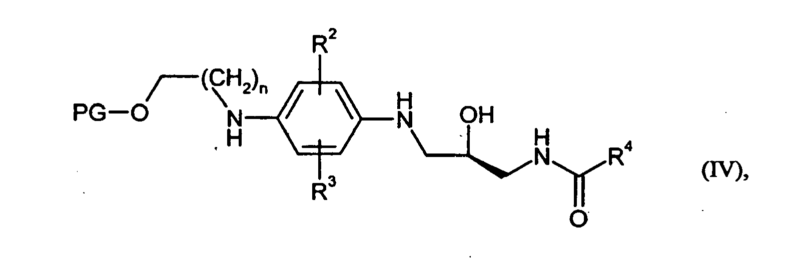 in welcher n, PG, R², R³ und R⁴ die oben angegebenen Bedeutungen aufweisen,
umsetzt, diese dann in einem inerten Lösungsmittel mit einem Kohlensäure-Äquivalent zu Verbindungen der Formel (V) 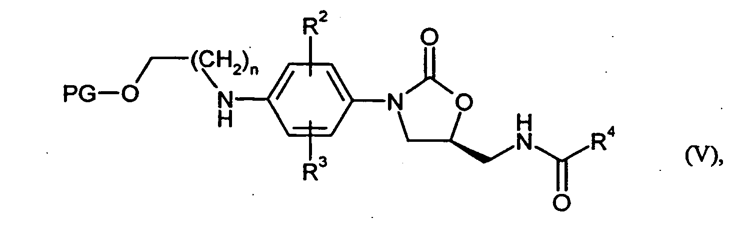 in welcher n, PG, R², R³ und R⁴ die oben angegebenen Bedeutungen aufweisen, reagiert,
anschließend entweder
[A] durch Abspaltung der Schutzgruppe PG zu Verbindungen der Formel (VI) 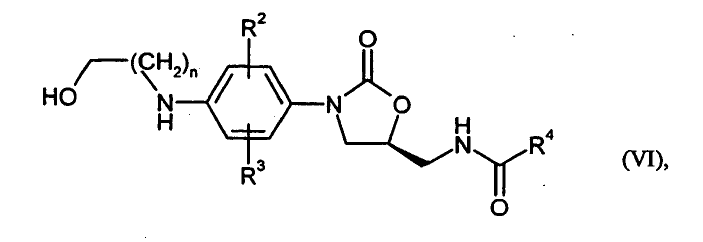 in welcher n, R², R³ und R⁴ die oben angegebenen Bedeutungen haben,
umsetzt und die Verbindungen der Formel (VI) dann in einem inerten Lösungsmittel in Gegenwart einer Säure mit Bromcyan in Verbindungen der Formel (I-A) 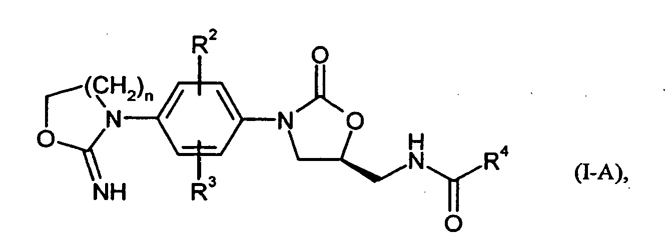 in welcher n, R², R³ und R⁴ die oben angegebenen Bedeutungen haben,
überführt
oder
[B] zuerst in einem inerten Lösungsmittel mit Bromcyan zu Verbindungen der Formel (VII) 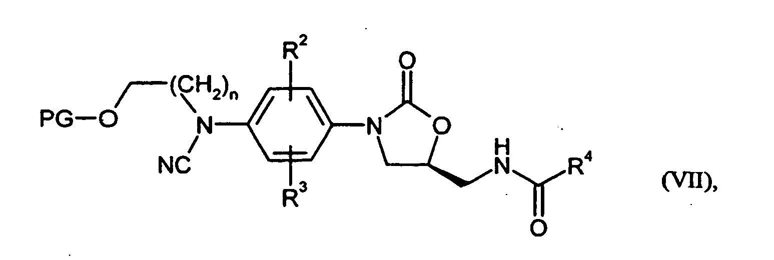 in welcher n, PG, R², R³ und R⁴ die oben angegebenen Bedeutungen haben, umsetzt,
anschließend durch Abspaltung der Schutzgruppe PG in Verbindungen der Formel (VIII) 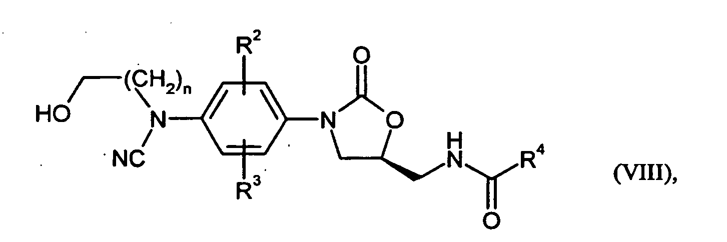 in welcher n, R², R³ und R⁴ die oben angegebenen Bedeutungen haben,
überführt und dann die Verbindungen der Formel (VIII) in einem inerten Lösungsmittel in Gegenwart einer Säure zu Verbindungen der Formel (I-A) cyclisiert
und die Verbindungen der Formel (I-A) gegebenenfalls mit den entsprechenden (i) Lösungsmitteln und/oder (ii) Basen oder Säuren zu ihren Solvaten, Salzen und/oder Solvaten der Salze umsetzt.

6. Verfahren zur Herstellung von Verbindungen der Formel (I), wie in Anspruch 1 definiert, in welcher R¹ für Wasserstoff und X für NH steht, **dadurch gekennzeichnet, dass** man ausgehend von Verbindungen der Formel (IV) 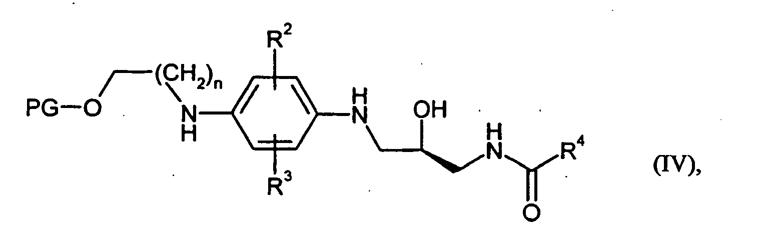 in welcher n, R², R³ und R⁴ die in Anspruch 1 angegebenen Bedeutungen aufweisen
und
PG für eine Hydroxy-Schutzgruppe steht,
zunächst durch Einführung einer zweiten Hydroxy-Schutzgruppe PG Verbindungen der Formel (IX) 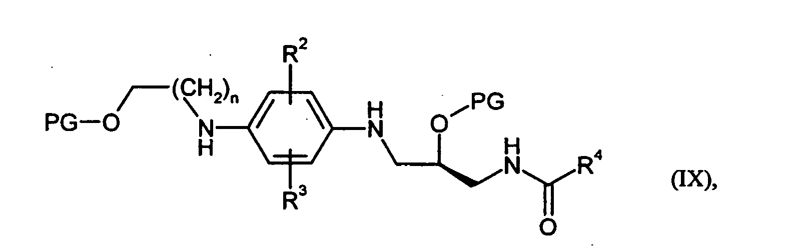 in welcher n, PG, R², R³ und R⁴ die oben angegebenen Bedeutungen aufweisen,
darstellt, diese dann in einem inerten Lösungsmittel mit Bromcyan in Verbindungen der Formel (X) 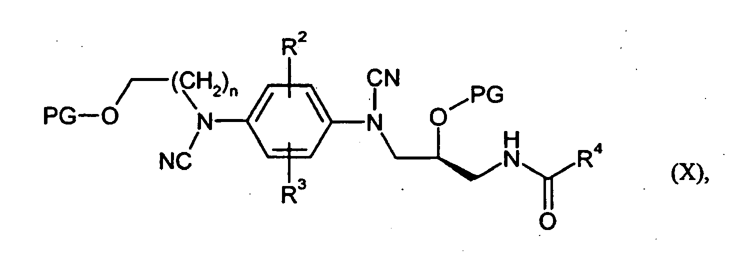 in welcher n, PG, R², R³ und R⁴ die oben angegebenen Bedeutungen haben,
überführt, anschließend durch Abspaltung der Schutzgruppen PG zu Verbindungen der Formel (XI) 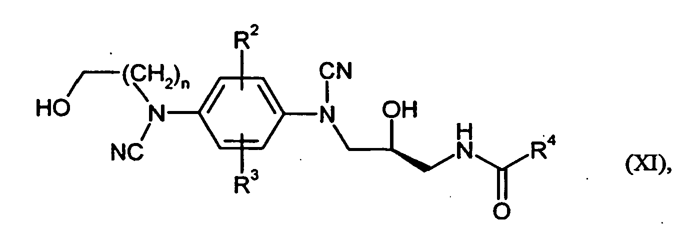 in welcher n, R², R³ und R⁴ die oben angegebenen Bedeutungen haben,
umsetzt und diese in einem inerten Lösungsmittel in Gegenwart einer Säure zu Verbindungen der Formel (I-B) 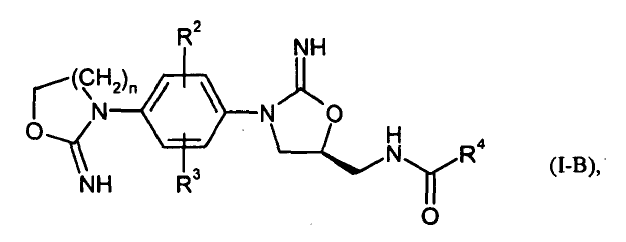 in welcher n, R², R³ und R⁴ die oben angegebenen Bedeutungen haben,
cyclisiert
und die Verbindungen der Formel (I-B) gegebenenfalls mit den entsprechenden (i) Lösungsmitteln und/oder (ii) Basen oder Säuren zu ihren Solvaten, Salzen und/oder Solvaten der Salze umsetzt.

7. Verbindung der Formel (I), wie in Anspruch 1 definiert, zur. Behandlung und/oder Prophylaxe von Krankheiten.

8. Verwendung einer Verbindung der Formel (I), wie in Anspruch 1 definiert, zur Herstellung eines Arzneimittels zur Behandlung und/oder Prophylaxe von thromboembolischen Erkrankungen.

9. Verwendung einer Verbindung der Formel (I), wie in Anspruch 1 definiert, zur Verhinderung der Blutkoagulation in vitro.

10. Arzneimittel enthaltend eine Verbindung der Formel (I), wie in Anspruch 1 definiert, in Kombination mit einem inerten, nicht-toxischen, pharmazeutisch geeigneten Hilfsstoff.

11. Arzneimittel enthaltend eine Verbindung der Formel (I), wie in Anspruch 1 definiert, in Kombination mit einem weiteren Wirkstoff.

12. Arzneimittel nach Anspruch 10 oder 11 zur Behandlung und/oder Prophylaxe von thromboembolischen Erkrankungen.

13. Verfahren zur Verhinderung der Blutkoagulation in vitro, **dadurch gekennzeichnet, dass** eine antikoagulatorisch wirksame Menge einer Verbindung der Formel (I), wie in Anspruch 1 definiert, zugegeben wird.

## Claims

1. Compound of the formula (I) 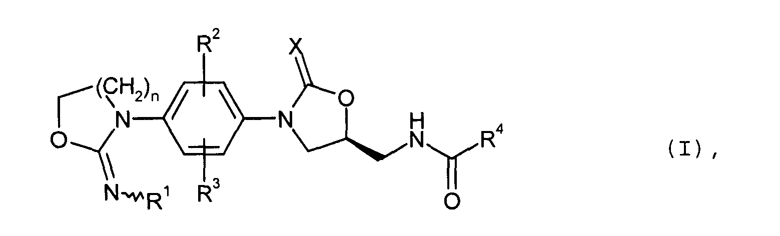 in which
R¹ represents hydrogen, hydroxyl, cyano, (C₁-C₆)- alkyl, (C₁-C₆)-alkanoyl, benzoyl or hetero- aroyl,
R² and R³ are identical or different and independently of one another represent hydrogen, fluorine, chlorine, cyano, (C₁-C₄)- alkyl, cyclopropyl, trifluoromethyl, hydroxyl, (C₁-C₄)-alkoxy, trifluoromethoxy, amino, mono- or di-(C₁-C₄)-alkylamino,
R⁴ represents phenyl, pyridyl, pyrimidinyl, pyrazinyl, thienyl, furyl or pyrrolyl which may in each case be mono- or disubstituted by identical or different substituents selected from the group consisting of halogen, cyano, (C₁-C₄) -alkyl, which for its part may be
n substituted by hydroxyl or amino, (C₁-C₄)- alkoxy, ethynyl, cyclopropyl and amino, represents the number 1, 2 or 3,
and
X represents O or N-R⁵, where
R⁵ represents hydrogen, cyano, (C₁-C₆)-alkyl or phenyl,
where phenyl may be mono- or disubstituted by identical or different substituents selected from the group consisting of halogen, cyano, trifluoromethyl, (C₁-C₄) -alkyl and (C₁-C₄)- alkoxy,
and salts, solvates and solvates of the salts thereof.

2. Compound of the formula (I) according to Claim 1 in which
R¹ represents hydrogen, hydroxyl, cyano, (C₁-C₆) - alkyl, (C₁-C₆)-alkanoyl, benzoyl or hetero- aroyl,
R² and R³ are identical or different and independently of one another represent hydrogen, fluorine, chlorine, cyano, (C₁-C₄)- alkyl, cyclopropyl, trifluoromethyl, hydroxyl, (C₁-C₄) -alkoxy, trifluoromethoxy, amino, mono- or di-(C₁-C₄)-alkylamino,
R⁴ represents phenyl, pyridyl, pyrimidinyl, pyrazinyl, thienyl, furyl or pyrrolyl which may in each case be mono- or disubstituted by identical or different substituents selected
from the group consisting of halogen, cyano, (C₁-C₄)-alkyl, which for its part may be substituted by hydroxyl or amino, (C₁-C₄)- alkoxy, ethynyl, cyclopropyl and amino,
n represents the number 1, 2 or 3,
and
X represents O or N-R⁵, where
R⁵ represents hydrogen, cyano or (C₁-C₆)-alkyl,
and salts, solvates and solvates of the salts thereof.

3. Compound of the formula (I) according to Claim 1 or 2 in which
R¹ represents hydrogen, hydroxyl, cyano or methyl,
R² represents hydrogen,
R³ represents hydrogen, fluorine, chlorine, cyano, methyl or methoxy,
R⁴ represents a group of the formula 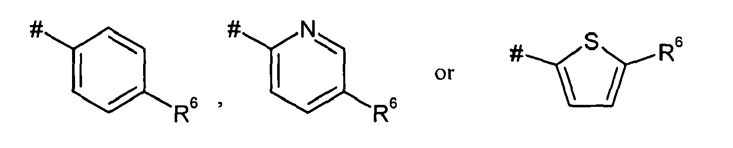 in which
R⁶ represents fluorine, chlorine, methyl or ethynyl
and
# denotes the point of attachment to the carbonyl group,
n represents the number 1 or 2,
and
X represents O,
and salts, solvates and solvates of the salts thereof.

4. Compound of the formula (I) according to any of Claims 1 to 3 in which
R¹ represents hydrogen,
R² represents hydrogen,
R³ represents hydrogen, fluorine or methyl,
R⁴ represents a group of the formula 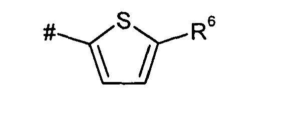 in which
R⁶ represents fluorine, chlorine or methyl
and
# denotes the point of attachment to the carbonyl group,
n represents the number 1 or 2,
and
X represents O, and salts, solvates and solvates of the salts thereof.

5. Process for preparing compounds of the formula (I), as defined in Claim 1, in which R¹ represents hydrogen and X represents oxygen, **characterized in that** compounds of the formula (II) 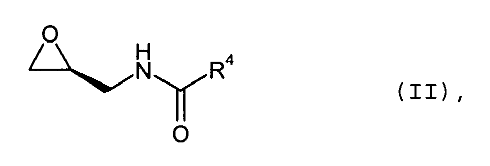 in which R⁴ has the meaning given in Claim 1,
are reacted in an inert solvent with a compound of the formula (III) 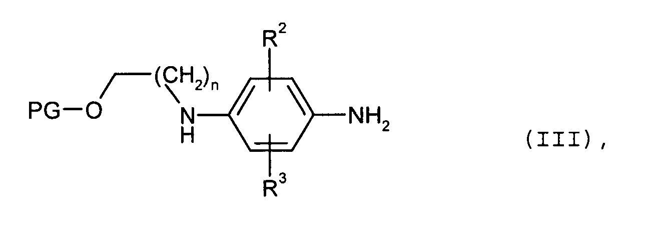 in which n, R² and R³ have the meanings given in Claim 1
and
PG represents a hydroxyl protective group,
to give compounds of the formula (IV) 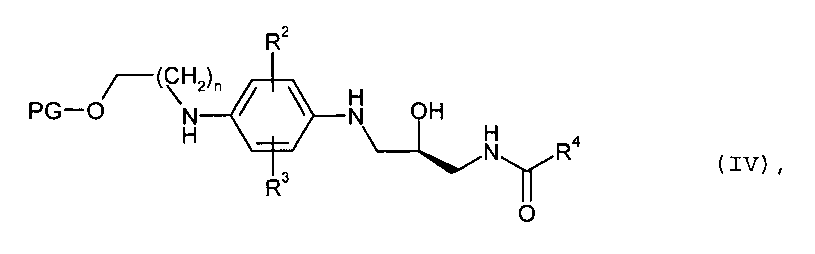 in which n, PG, R², R³ and R⁴ have the meanings given above,
these are then reacted in an inert solvent with a carbonic acid equivalent to give compounds of the formula (V) 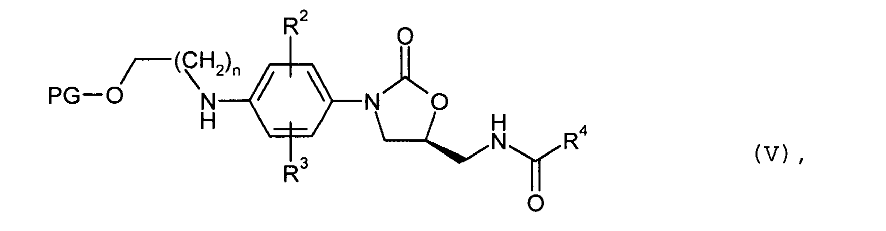 in which n, PG, R², R³ and R⁴ have the meanings given above,
and then either
[A] by removal of the protective group PG converted into compounds of the formula (VI) 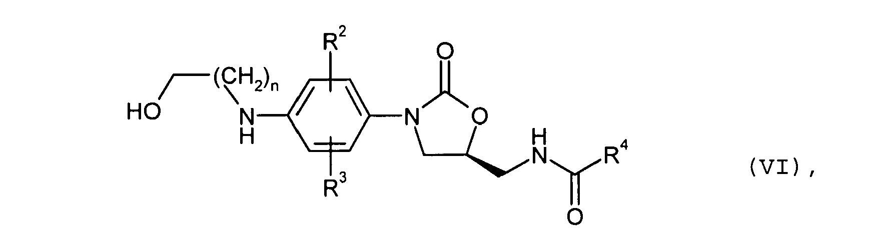 in which n, R², R³ and R⁴ have the meanings given above,
and the compounds of the formula (VI) are then in an inert solvent in the presence of an acid converted with cyanogen bromide into compounds of the formula (I-A) 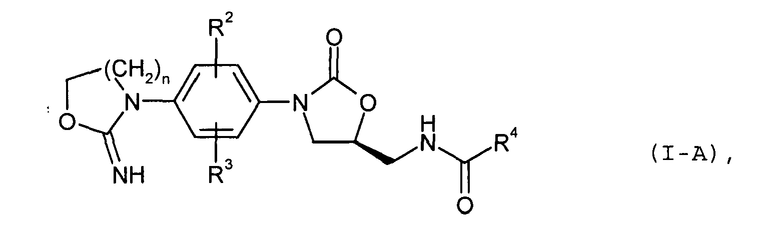 in which n, R², R³ and R⁴ have the meanings given above,
or
[B] initially reacted in an inert solvent with cyanogen bromide to give compounds of the formula (VII) 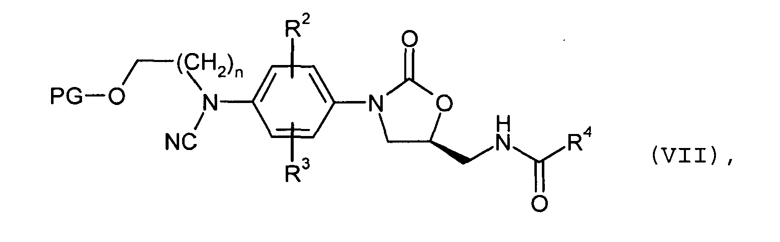 in which n, PG, R², R³ and R⁴ have the meanings given above,
then by removal of the protective group PG converted into compounds of the formula (VIII) 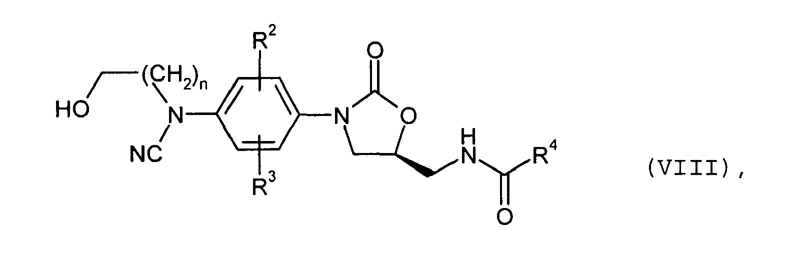
in which n, R², R³ and R⁴ have the meanings given above,
and the compounds of the formula (VIII) are then cyclized in an inert solvent in the presence of an acid to give compounds of the formula (I-A)
and the compounds of the formula (I-A) are, if appropriate, converted with the appropriate (i) solvents and/or (ii) bases or acids into their solvates, salts and/or solvates of the salts.

6. Process for preparing compounds of the formula (I), as defined in Claim 1, in which R¹ represents hydrogen and X represents NH, **characterized in that**, starting with compounds of the formula (IV) 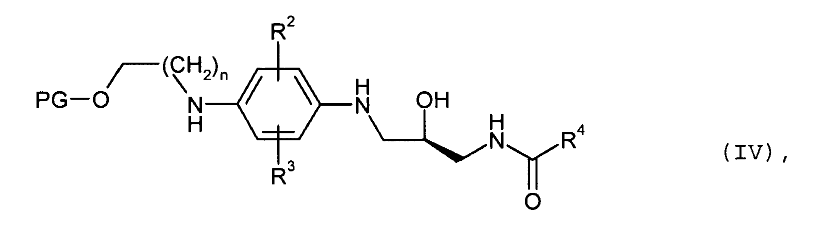 in which n, R², R³ and R⁴ have the meanings given in Claim 1
and
PG represents a hydroxyl protective group,
initially by introducing a second hydroxyl protective group PG, compounds of the formula (IX) 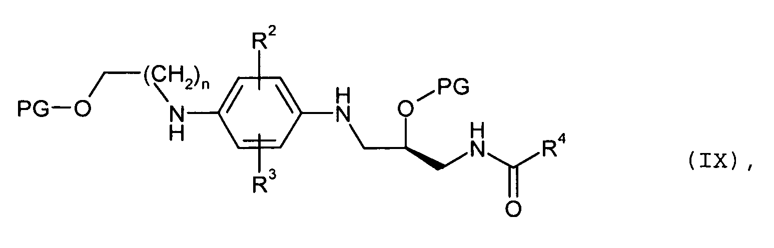 in which n, PG, R², R³ and R⁴ have the meanings given above,
are prepared, which are then, in an inert solvent using cyanogen bromide, converted into compounds of the formula (X) 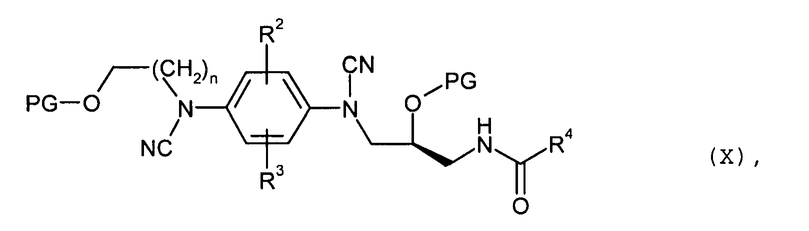 in which n, PG, R², R³ and R⁴ have the meanings given above,
then, by removing the protective groups PG, converted into compounds of the formula (XI) 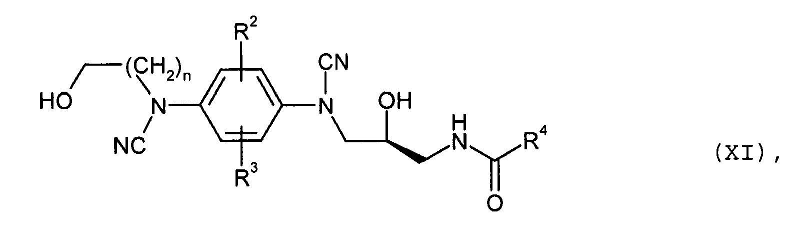 in which n, R², R³ and R⁴ have the meanings given above,
and these are cyclized in an inert solvent in the presence of an acid to compounds of the formula (I-B) 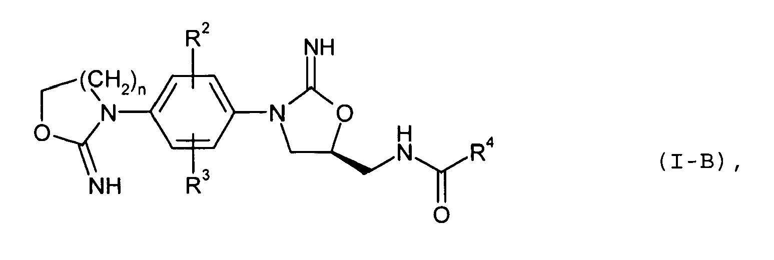 in which n, R², R³ and R⁴ have the meanings given above,
and the compounds of the formula (I-B) are, if appropriate, converted with the appropriate (i) solvents and/or (ii) bases or acids into their solvates, salts and/or solvates of the salts.

7. Compound of the formula (I) as defined in Claim 1 for the treatment and/or prophylaxis of diseases.

8. Use of a compound of the formula (I) as defined in Claim 1 for preparing a medicament for the treatment and/or prophylaxis of thromboembolic disorders.

9. Use of a compound of the formula (I) as defined in Claim 1 for preventing blood coagulation in vitro.

10. Medicament, comprising a compound of the formula (I) as defined in Claim 1 in combination with an inert nontoxic, pharmaceutically acceptable auxiliary.

11. Medicament, comprising a compound of the formula (I) as defined in Claim 1 in combination with a further active compound.

12. Medicament according to Claim 10 or 11 for the treatment and/or prophylaxis of thromboembolic disorders.

13. Method for preventing blood coagulation in vitro, **characterized in that** an anticoagulatorilly effective amount of a compound of the formula (I) as defined in Claim 1 is added.

## Revendications

1. Composé de formule (I) 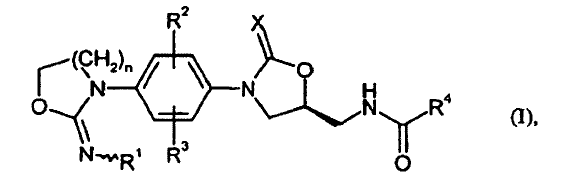 dans laquelle
R¹ représente hydrogène, hydroxy, cyano, (C₁-C₆)- alkyle, (C₁-C₆)-alcanoyle, benzoyle ou hétéroaroyle,
R² et R³ sont identiques ou différents et représentent, indépendamment l'un de l'autre, hydrogène, fluor, chlore, cyano, (C₁-C₄)-alkyle, cyclopropyle, trifluorométhyle, hydroxy, (C₁-C₄) -alcoxy, trifluorométhoxy, amino, mono-(C₁-C₄)-alkylamino ou di-(C₁-C₄-alkylamino,
R⁴ représente phényle, pyridyle, pyrimidinyle, pyrazinyle, thiényle, furyle ou pyrrolyle, qui peuvent à chaque fois être monosubstitués ou disubstitués, de manière identique ou différente, par des substituants choisis dans le groupe formé par halogène, cyano, (C₁-C₄)-alkyle, qui peut à son tour être substitué par hydroxy ou amino ; (C₁-C₄)- alcoxy, éthynyle, cyclopropyle et amino,
n représente le nombre 1, 2 ou 3 et
X représente O ou N-R⁵, où
R⁵ signifie hydrogène, cyano, (C₁-C₆)-alkyle ou phényle,
où phényle peut être monosubstitué ou disubstitué, de manière identique ou différente, par des substituants choisis dans le groupe formé par halogène, cyano, trifluorométhyle, (C₁-C₄)-alkyle et (C₁-C₄)-alcoxy,
ainsi que ses sels, solvates et les solvates des sels.

2. Composé de formule (I) selon la revendication 1, dans laquelle
R¹ représente hydrogène, hydroxy, cyano, (C₁-C₆)- alkyle, (C₁-C₆)-alcanoyle, benzoyle ou hétéroaroyle,
R² et R³ sont identiques ou différents et représentent, indépendamment l'un de l'autre, hydrogène, fluor, chlore, cyano, (C₁-C₄)-alkyle, cyclopropyle, trifluorométhyle, hydroxy, (C₁-C₄)-alcoxy, trifluorométhoxy, amino, mono-(C₁-C₄)-alkylamino ou di- (C₁-C₄) -alkylamino,
R⁴ représente phényle, pyridyle, pyrimidinyle, pyrazinyle, thiényle, furyle ou pyrrolyle, qui peuvent à chaque fois être monosubstitués ou disubstitués, de manière identique ou différente, par des substituants choisis dans le groupe formé par halogène, cyano, (C₁-C₄)-alkyle, qui peut à son tour être substitué par hydroxy ou amino; (C₁-C₄)- alcoxy, éthynyle, cyclopropyle et amino,
n représente le nombre 1, 2 ou 3 et
X représente O ou N-R⁵, où R⁵ signifie hydrogène, cyano ou (C₁-C₆)-alkyle,
ainsi que ses sels, solvates et les solvates des sels.

3. Composé de formule (I) selon l'une quelconque des revendications 1 ou 2, dans laquelle
R¹ représente hydrogène, hydroxy, cyano ou méthyle,
R² représente hydrogène,
R³ représente hydrogène, fluor, chlore, cyano, méthyle ou méthoxy,
R⁴ représente un groupe de formule 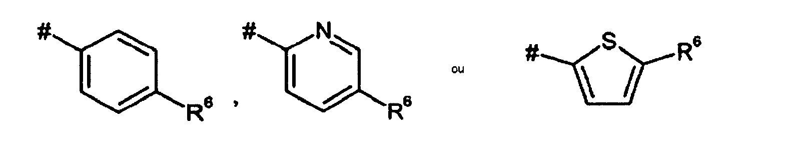 où
R⁶ signifie fluor, chlore, méthyle ou éthynyle et
# signifie le site de liaison avec le groupe carbonyle,
n représente le nombre 1 ou 2, et
X représente O ;
ainsi que ses sels, solvates et les solvates des sels.

4. Composé de formule (I) selon l'une quelconque des revendications 1 à 3, dans laquelle
R¹ représente hydrogène,
R² représente hydrogène,
R³ représente hydrogène, fluor ou méthyle,
R⁴ représente un groupe de formule 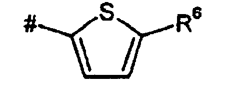 où
R⁶ signifie fluor, chlore ou méthyle et
# signifie le site de liaison avec le groupe carbonyle,
n représente le nombre 1 ou 2, et
X représente O ;
ainsi que ses sels, solvates et les solvates des sels.

5. Procédé pour la préparation de composés de formule (I), tels que définis dans la revendication 1, dans laquelle R¹ représente hydrogène et X représente oxygène, **caractérisé en ce qu'**on transforme des composés de formule (II) 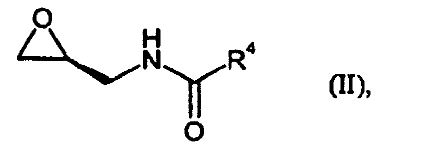 dans laquelle R⁴ présente la signification indiquée dans la revendication 1,
dans un solvant inerte avec un composé de formule (III) 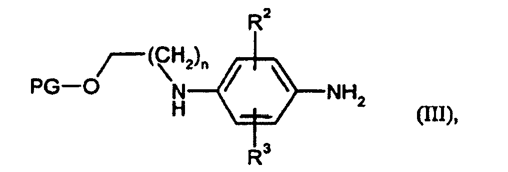 dans laquelle n, R² et R³ ont les significations indiquées dans la revendication 1 et
PG représente un groupe de protection de la fonction hydroxy,
en composés de formule (IV) 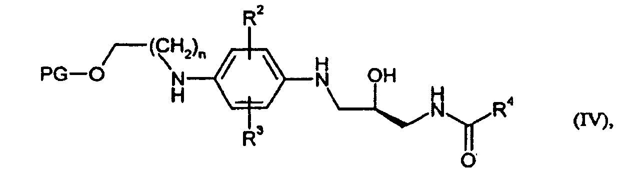 dans laquelle n, PG, R², R³ et R⁴ présentent les significations indiquées ci-dessus,
on fait ensuite réagir ceux-ci dans un solvant inerte avec un équivalent d'acide carbonique en composés de formule (V) 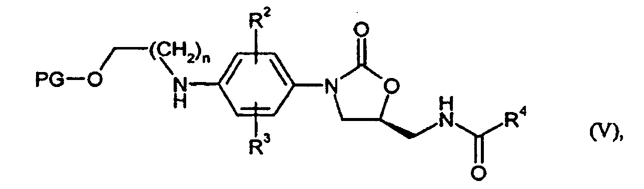 dans laquelle n, PG, R², R³ et R⁴ présentent les significations indiquées ci-dessus,
ensuite, on transforme ceux-ci soit
[A] par dissociation du groupe de protection PG en composés de formule (VI) 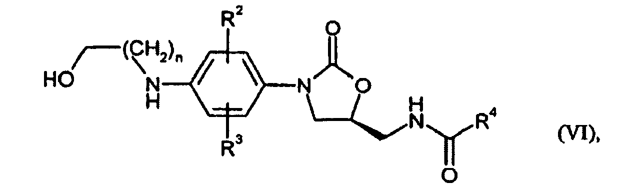 dans laquelle n, R², R³ et R⁴ ont les significations indiquées ci-dessus,
et on transforme alors les composés de formule (VI) dans un solvant inerte en présence d'un acide avec du bromocyan en composés de formule (I-A) 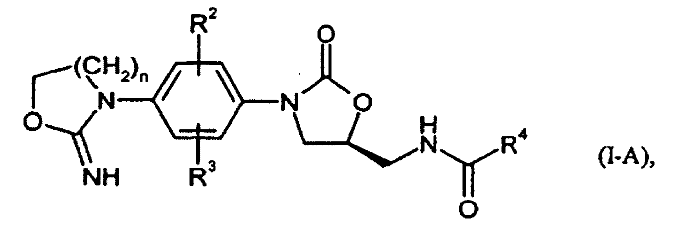 dans laquelle n, R², R³ et R⁴ ont les significations indiquées ci-dessus,
soit
[B] d'abord dans un solvant inerte avec du bromocyan en composés de formule (VII) 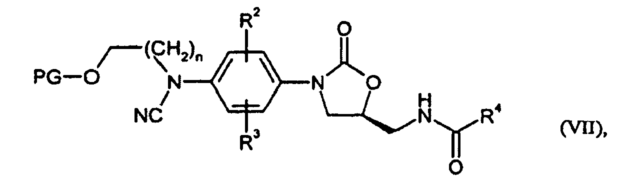 dans laquelle n, PG, R², R³ et R⁴ ont les significations indiquées ci-dessus,
puis on transforme ceux-ci par dissociation du groupe de protection PG en composés de formule (VIII) 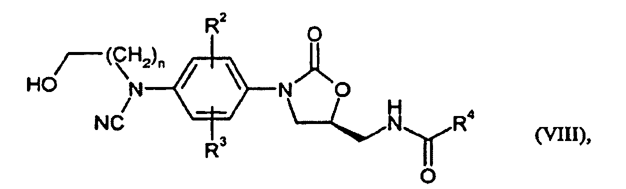
dans laquelle n, R², R³ et R⁴ ont les significations indiquées ci-dessus,
et on cyclise alors les composés de formule (VIII) dans un solvant inerte en présence d'un acide en composés de formule (I-A)
et on transforme les composés de formule (I-A) le cas échéant avec (i) les solvants et/ou (ii) les bases ou acides correspondants en leurs solvates, sels et/ou les solvates des sels.

6. Procédé pour la préparation de composés de formule (I), tels que définis dans la revendication 1, dans laquelle R¹ représente hydrogène et X représente NH, **caractérisé en ce qu'**on prépare d'abord, partant des composés de formule (IV) 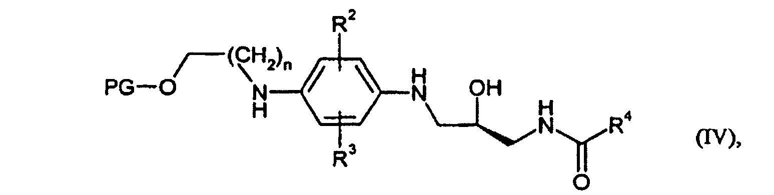 dans laquelle n, R², R³ et R⁴ ont les significations indiquées dans la revendication 1 et
PG représente un groupe de protection de la fonction hydroxy,
par introduction d'un deuxième groupe de protection de la fonction hydroxy PG, des composés de formule (IX) 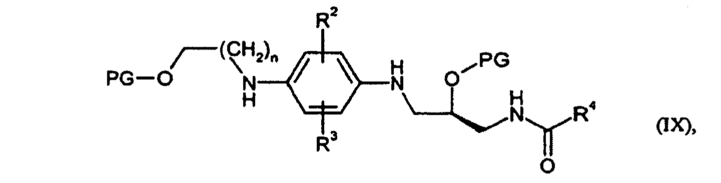 dans laquelle n, PG, R², R³ et R⁴ présentent les significations indiquées ci-dessus,
on transforme ensuite ceux-ci alors dans un solvant inerte avec du bromocyan en composés de formule (X) 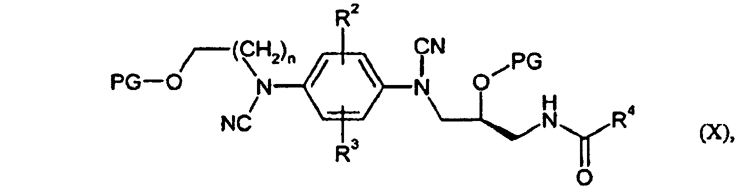 dans laquelle n, PG, R², R³ et R⁴ ont les significations indiquées ci-dessus,
puis on transforme ceux-ci par dissociation des groupes de protection PG en composés de formule (XI) 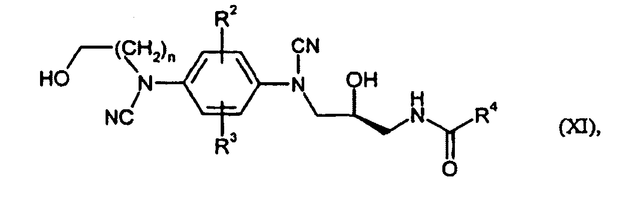 dans laquelle n, R², R³ et R⁴ ont les significations indiquées ci-dessus,
et on cyclise ceux-ci dans un solvant inerte en présence d'un acide en composés de formule (I-B) 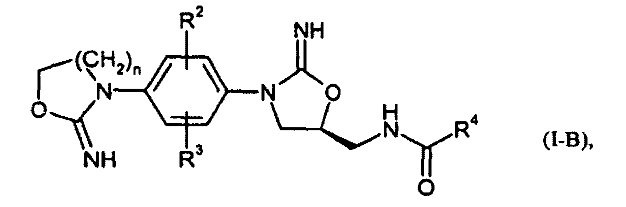 dans laquelle n, R², R³ et R⁴ ont les significations indiquées ci-dessus,
et on transforme les composés de formule (I-B) le cas échéant avec (i) les solvants et/ou (ii) les bases ou acides correspondants en leurs solvates, sels et/ou solvates des sels.

7. Composé de formule (I), tel que défini dans la revendication 1, destiné au traitement et/ou à la prophylaxie de maladies.

8. Utilisation d'un composé de formule (I), tel que défini dans la revendication 1, pour la préparation d'un médicament destiné au traitement et/ou à la prophylaxie de maladies thromboemboliques.

9. Utilisation d'un composé de formule (I), tel que défini dans la revendication 1, destinée à empêcher la coagulation du sang in vitro.

10. Médicament contenant un composé de formule (I), tel que défini dans la revendication 1, en combinaison avec un adjuvant inerte, non toxique, pharmaceutiquement approprié.

11. Médicament contenant un composé de formule (I), tel que défini dans la revendication 1, en combinaison avec une autre substance active.

12. Médicament selon la revendication 10 ou 11 destiné au traitement et/ou à la prophylaxie de maladies thromboemboliques.

13. Procédé destiné à empêcher la coagulation du sang in vitro, **caractérisé en ce qu'**on ajoute une quantité active en anticoagulation d'un composé de formule (I), tel que défini dans la revendication 1.
